(19) Europäisches Patentamt
European Patent Office
Office européen des brevets

(11) Veröffentlichungsnummer: **0 243 905**
**B1**

# (12) EUROPÄISCHE PATENTSCHRIFT

(45) Veröffentlichungstag der Patentschrift: **10.10.90**

(51) Int. Cl.[5]: **C 07 D 295/08**, A 61 K 31/495

(21) Anmeldenummer: **87106034.9**

(22) Anmeldetag: **28.04.87**

## (54) 1(omega-[bis-(Phenyl)-alkoxy]-alkyl)-4-(di-(phenyl)-methyl)-Piperazine, Verfahren zu ihrer Herstellung und diese enthaltende Arzneimittel.

(30) Priorität: **28.04.86 HU 175186**

(43) Veröffentlichungstag der Anmeldung:
**04.11.87 Patentblatt 87/45**

(45) Bekanntmachung des Hinweises auf die Patenterteilung:
**10.10.90 Patentblatt 90/41**

(84) Benannte Vertragsstaaten:
**AT BE CH DE FR GB IT LI LU NL SE**

(56) Entgegenhaltungen:
**FR-A-2 374 318**

**BELGISCHE CHEMISCHE INDUSTRIE, Band T.XIX, Nr. 11, 1954, Seiten 1176-1196; H. MORREN et al.: "Nouveaux dérivés 1.4-disubstitués de la Pipérazine; Etude de leurs propriétés antihistaminiques"**

(73) Patentinhaber: **Richter Gedeon Vegyészeti Gyár R.T.**
**Gyömröi ut 19-21**
**H-1475 Budapest X (HU)**

(72) Erfinder: **Tóth, Edit**
**Szabolcska M. u. 7**
**H-1114 Budapest (HU)**
Erfinder: **Kiss, Béla**
**Vöröshadsereg utja 197/c**
**H-2220 Vecsés (HU)**
Erfinder: **Törley, József**
**Katona J. u. 41**
**H-1137 Budapest (HU)**
Erfinder: **Pálosi, Eva, Dr.**
**Vend u. 21**
**H-1025 Budapest (HU)**
Erfinder: **Hajdu, István**
**Tátra tér B/4**
**H-1205 Budapest (HU)**
Erfinder: **Szporny, László, Dr.**
**M. u. 7**
**H-1114 Szabolcska (HU)**
Erfinder: **Groó, Dóra, Dr.**
**Napraforgó u. 17**
**H-1021 Budapest (HU)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Courier Press, Leamington Spa, England.

(72) Erfinder: Lapis, Erzsébet, Dr.
Abaliget u. 86
H-1172 Budapest (HU)
Erfinder: Laszlovszky, István, Dr.
Bartók B. u. 16
H-1111 Budapest (HU)

(74) Vertreter: Beszédes, Stephan G., Dr.
Patentanwalt
Münchener Strasse 80a Postfach 1168
D-8060 Dachau (DE)

**Beschreibung**

Die Erfindung betrifft neue 1-{ω-[bis-(Phenyl)-alkoxy]-alkyl}-4-{di-(phenyl)-methyl}-piperazine, ein Verfahren zu ihrer Herstellung und diese Verbindungen enthaltende Arzneimittel, insbesondere mit dopaminerger Wirkung auf das Zentralnervensystem.

Aus dem Schrifttum sind zahlreiche therapeutisch wirksame 1,4-disubstituierte Piperazinderivate, bei welchen das eine Stickstoffatom des Piperazines durch einen Benzhydryl- beziehungsweise Benzhydryloxyalkylrest substituiert ist und am anderen Stickstoffatom dioe verschiedensten Gruppen hängen, bekannt. Solche Verbindungen sind beispielsweise in den belgischen Patentschriften 523 899, 523 900 bis 523 903 und 649 848, in der holländischen Patentschrift 8 202 636, in der britischen Patentschrift 809 760, in den deutschen Patentschriften 2 719 146 und 2 755 752, in der japanischen Patentschrift 7 411 713, in der südafrikanischen Patentschrift 6 906 642 und in der US-Patentschrift 3 178 422 beschrieben.

In der Literaturstelle "Belgische Chemische Industrie", Band T. XIX, Nr. 11, 1954 werden auf den Seiten 1176—1196 1,4-disubstituierte Piperazinderivate beschrieben, die eine Antihistaminwirkung besitzen.

Die FR—A—2 374 318 beschreibt Piperazinderivate mit einer von den erfindungsgemäßen Verbindungen verschiedenen Struktur, die eine dopaminerge Wirkung zeigen.

Der Erfindung liegt die Aufgabe zugrunde, überlegene pharmakologische Wirkungen aufweisende neue 1,4-disubstituierte Piperazinderivate, ein Verfahren zu ihrer Herstellung und diese Verbindungen enthaltende Arzneimittel zu schaffen.

Das Obige wurde überraschenderweise durch die Erfindung erreicht.

Gegenstand der Erfindung sind 1-{ω-[bis-(Phenyl)-alkoxy]-alkyl}-4-{di-(phenyl)-methyl}-piperazine der allgemeinen Formel

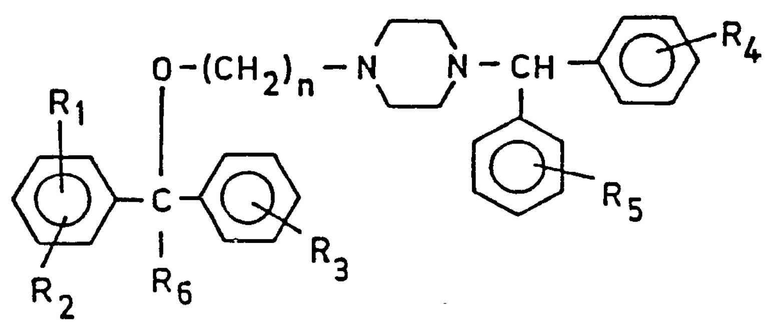

I ,

worin

$R_1$, $R_2$, $R_3$, $R_4$ und $R_5$, die gleich oder verschieden sein können, für Wasserstoffatome, Halogenatome, Trihalogenmethylreste, Alkylreste mit 1 bis 4 Kohlenstoffatom(en) oder Alkoxyreste mit 1 bis 4 Kohlenstoffatom(en) stehen,

$R_6$ ein Wasserstoffatom oder einen Alkylrest mit 1 bis 4 Kohlenstoffatom(en) bedeutet und

n 2 oder 3 ist,

sowie ihre Säureadditionssalze und quaternären Salze und optisch aktiven Isomere und Solvate.

Die erfindungsgemäßen 1-{ω-[bis-(Phenyl)-alkoxy]-alkyl}-4-{di-(phenyl)-methyl}-piperazine können 1 oder mehrere asymmetrische Kohlenstoffatome haben und daher in verschiedenen Stereoisomerformen vorkommen. Sie können also Basen, Säureadditionssalze, quarternäre Salze, insbesondere quaternäre Ammoniumsalze, Racemate, getrennte optische Isomere und deren Gemische sowie Solvate, zum Beispiel Hydrate, sein.

Die erfindungsgemäßen 1-{ω-[bis-(Phenyl)-alkoxy]-alkyl}-4-{di-(phenyl)-methyl}-piperazine unterscheiden sich von den bekannten Verbindungen darin, daß sie die Benzhydryloxyalkylgruppe im Molekül aufweisen. Aus dem Schrifttum ist hingegen keine einzige Verbindung dieses Typs mit einer Benzhydrylgruppe am anderen Stickstoffatom des Piperazinringes bekannt.

Vorzugsweise ist beziehungsweise sind das beziehungsweise die Halogenatom(e), für welche[s] $R_1$, $R_2$, $R_3$, $R_4$ und/oder $R_5$ stehen kann beziehungsweise können, und/oder die Halogenatome des beziehungsweise der Trihalogenmethylreste[s], für welche[n] $R_1$, $R_2$, $R_3$, $R_4$ und/oder $R_5$ stehen kann beziehungsweise können, Fluor und/oder Chlor.

Es ist auch bevorzugt, daß der beziehungsweise die Alkyl- und/oder Alkoxyrest(e), für welche[n] $R_1$, $R_2$, $R_3$, $R_4$ und/oder $R_5$ stehen kann beziehungsweise können, [ein] solche[r] mit 1 oder 2, insbesondere 1, Kohlenstoffatom[en] ist beziehungsweise sind.

Ferner ist es bevorzugt, daß der Alkylrest, für den $R_6$ stehen kann, ein solcher mit 1 bis 3, insbesondere 1 oder 3, Kohlenstoffatom(en) ist.

Weiterhin ist es bevorzugt, daß der beziehungsweise die an einem oder mehreren Phenylring(en) gegebenenfalls vorliegende(n) Substituent(en) in der beziehungsweise den 3- und/oder 4-Stellung(en) ist beziehungsweise sind.

Besonders bevorzugte erfindungsgemäße 1-{ω-[bis-(Phenyl)-alkoxy]-alkyl}-4-{di-(phenyl)-methyl}-piperazine sind

1-[3'-⟨bis-{4''-(Fluor)-phenyl}-methoxy⟩-propyl]-4-[bis-{4'''-(fluor)-phenyl}-methyl]-piperazin,

1-[3'-⟨{4''-(Methoxy)-phenyl}-{3'''-(trifluormethyl)-phenyl}-methoxy⟩-propyl]-4-[{4''''-(methoxy)-phenyl}-{3'''''-(trifluormethyl)-phenyl}-methyl]-piperazin,

1-[3'-⟨bis-{4''-(Fluor)-phenyl}-methoxy⟩-propyl]-4-{2'''-(chlor)-phenyl}-{4''''-(fluor)-phenyl}-methyl]-piperazin,

1-[3'-⟨Di-(phenyl)-methoxy⟩-propyl]-4-[{2''-(chlor)-phenyl}-{4'''-(fluor)-phenyl}-methyl]-piperazin,

1-[2'⟨bis-{4''-(Fluor)-phenyl}-methoxy⟩-äthyl]-4-[di-(phenyl)-methyl]-piperazin,

1-[3'-⟨bis-{4''-(Fluor)-phenyl}-methoxy⟩-propyl]-4-[{4'''-(fluor)-phenyl}-{phenyl}-methyl]-piperazin,

1-[3'-⟨{Di-(phenyl)}-methoxy⟩-propyl]-4-[bis-{fluorphenyl}-methyl]-piperazin und 1-[3'-⟨{Di-(phenyl)}-methoxy⟩-propyl]-4-[{4''-(fluor)-phenyl}-{phenyl}-methyl]-piperazin

sowie ihre Säureadditionssalze und quaternären Salze.

Die Säureadditionssalze können solche mit anorganischen oder organischen Säuren sein. Zweckmäßig sind sie solche mit physiologisch brauchbaren Säuren. Beispiele für solche sind Halogenwasserstoffsäuren, wie Salzsäure, und Bromwasserstoffsäure, Schwefelsäure, Phosphorsäuren, Ameisensäure, Essigsäure, Propionsäure, Oxalsäure, Glykolsäure, Maleinsäure, Fumarsäure, Bernsteinsäure, Weinsäure, Ascorbinsäure, Citronensäure, Apfelsäure, Salicylsäure, Milchsäure, Benzoesäure, Zimtsäure, Asparaginsäure, Glutaminsäure, N-(Acetyl)-asparaginsäure, N-(Acetyl)-glutaminsäure, Alkylsulfonsäuren, wie Methansulfonsäure, und Arylsulfonsäuren, wie Toluolsulfonsäure.

Die quaternären Salze sind vorzugsweise quaternäre Ammoniumsalze. Zweckmäßig sind sie solche mit physiologisch brauchbaren Quaternisierungsmitteln. Beispiele für solche sind niedere Alkyl- und Alkenylhalogenide und Benzylhalogenide und niedere Alkylsulfate.

Gegenstand der Erfindung ist auch ein Verfahren zur Herstellung der erfindungsgemäßen Verbindungen, welches dadurch gekennzeichnet ist, daß

a) Benzhydrylhalogenide, Benzhydrole beziehungsweise ihre Alkoholate der allgemeinen Formel

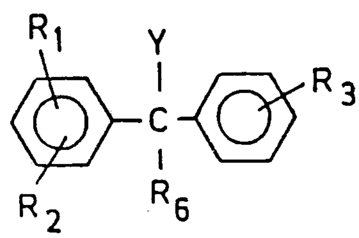

II ,

worin

$R_1$, $R_2$, $R_3$ und $R_6$ die oben angegebenen Bedeutungen haben und

Y für ein Halogenatom, eine Hydroxygruppe oder einen Rest der allgemeinen Formel —OM, in welchletzterer M ein Alkalimetallatom oder einen Rest der Formel —MgHlg in welchletzterer Hlg ein Halogenatom ist, bedeutet, steht, mit 1-[Di-(phenyl)-methyl]-piperazin-4-ylalk-ω-ylhalogeniden, -4-ylalkan-ω-olen beziehungsweise -4-ylalk-ω-yl-aten der allgemeinen Formel

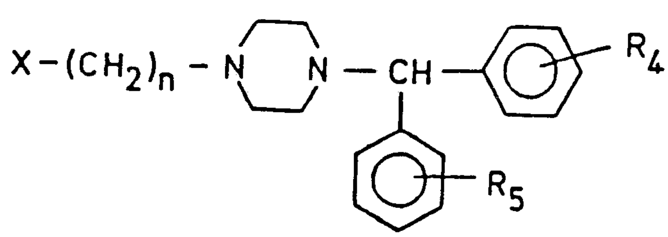

III ,

worin

$R_4$, $R_5$ und n die oben angegebenen Bedeutungen haben und

X für ein Halogenatom, eine Hydroxygruppe oder einen Rest der allgemeinen Formel —OM', in welchletzterer M' ein Alkalimetallatom bedeutet, steht,

mit der Einschränkung, daß

α) X eine Hydroxygruppe oder einen Rest der allgemeinen Formel —OM' bedeutet, wenn Y für ein Halogenatom steht oder

β) X eine Hydroxygruppe oder ein Halogenatom bedeutet, wenn Y für eine Hydroxygruppe steht, oder

γ) X ein Halogenatom bedeutet, wenn Y für einen Rest der allgemei-Formel —OM steht,

in einem wasserfreien, für die Reaktion neutralen organischen Lösungsmittel umgesetzt werden, wobei für den Fall, daß von den Substituenten X und Y der eine eine Hydroxylgruppe und der andere ein Halogenatom ist, die Reaktion in Gegenwart einer zur Bindung der bei der Reaktion freiwerdenden Säure

geeigneten anorganischen oder organischen Base durchgeführt wird, und für den Fall, daß sowohl X als auch Y für eine Hydroxylgruppe stehen, die Kondensation in Gegenwart von bei der Ätherbildung gebräuchlichen anorganischen oder organischen Säuren oder ihren sauren Salzen durchgeführt und das entstehende Wasser azeotropisch herausdestilliert wird, oder

b) [Di-(phenyl)]-[alkylbeziehungsweise arylsulfonyloxyalk-ω-oxy]-alkane beziehungsweise [Di-(phenyl)-[halogenalk-ω-oxy]alkane der allgemeinen Formel

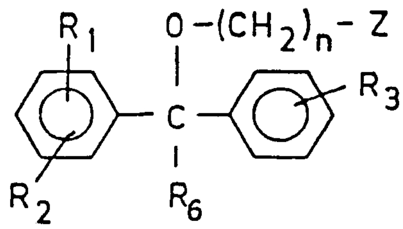

IV ,

worin

$R_1$, $R_2$, $R_3$, $R_6$ und n die oben angegebenen Bedeutungen haben und

Z für einen Alkyl- oder Arylsulfonyloxyrest oder ein Halogenatom steht,

mit 1-(Benzhydryl)-piperazinen der allgemeinen Formel

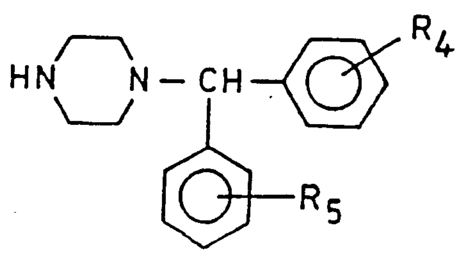

V ,

worin

$R_4$ und $R_5$ die oben angegebenen Bedeutungen haben,

in einem inerten organischen Lösungsmittel in Gegenwart einer zur Bindung der bei der Reaktion freiwerdenden Säure geeigneten Base umgesetzt werden oder

c) 1-{ω-[bis-(Phenyl)-alkoxy]-alkyl}-piperazine der allgemeinen Formel

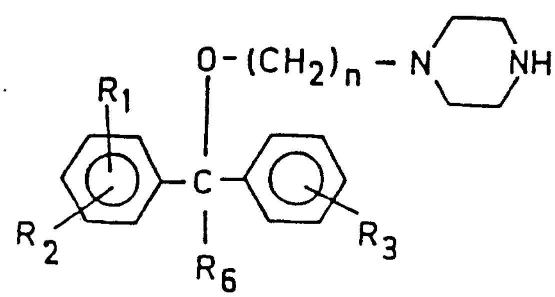

VI ,

worin

$R_1$, $R_2$, $R_3$, $R_6$ und n die oben angegebenen Bedeutungen haben, mit Benzhydrylhalogeniden der allgemeinen Formel

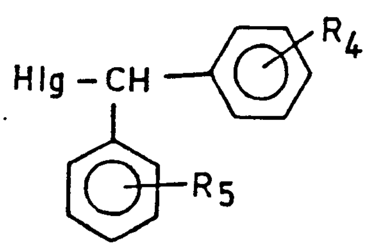

VII ,

worin

$R_4$ und $R_5$ die oben angegebenen Bedeutungen haben und Hlg die oben angegebene Bedeutung hat, unter den vorstehend unter b) beschriebenen Bedingungen umgesetzt werden oder

d) 1-{ω-[bis-(Phenyl)-alkoxy]-alkanoyl}-4-{di-(phenyl)-methyl}-piperazine der allgemeinen Formel

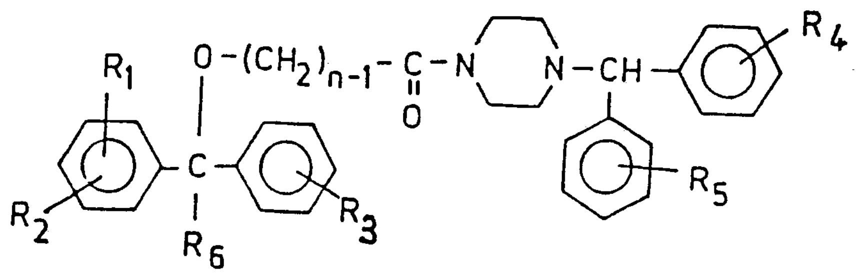

VIII ,

worin

$R_1$, $R_2$, $R_3$, $R_4$, $R_5$, $R_6$ und n die oben angegebenen Bedeutungen haben, in einem inerten organischen Lösungsmittel unter Verwendung eines Aluminiumkomplexes reduziert werden

sowie in an sich bekannter Weise gegebenenfalls die nach einer der Varianten a) bis d) erhaltenen 1-{ω-[bis-(Phenyl)-alkoxy]-alkyl}-4-{di-(phenyl)-methyl}-piperazine der allgemeinen Formel I mit anorganischen oder organischen Säuren in Säureadditionssalze oder mit Quaternisierungsmitteln in quaternäre Salze überführt und/oder die erhaltenen Säureadditionssalze oder quaternären Salze der 1-{ω-[bis-(Phenyl)-alkoxy]-alkyl}-4-{di-(phenyl)-methyl}-piperazine der allgemeinen Formel I in die entsprechenden freien 1-{ω-[bis-(Phenyl)-alkoxy]-alkyl}-4-{di-(phenyl)-methyl}-piperazinbasen der allgemeinen Formel I oder in andere Säureadditionssalze und/oder quaternäre Salze überführt werden und/oder gegebenenfalls eine Spaltung der erhaltenen racemischen 1-{ω-[bis-(Phenyl)-alkoxy]-alkyl}-4-{di-(phenyl)-methyl}-piperazine der allgemeinen Formel I beziehungsweise von deren Säureadditionssalzen beziehungsweise quaternären Salzen beziehungsweise eine Racemisierung der entsprechenden optisch aktiven Verbindungen in Stereoisomere vorgenommen wird.

Als Alkalimetall, für welches M beziehungsweise M' stehen kann, wird bevorzugt Lithium, Kalium oder Natrium verwendet.

Sofern im erfindungsgemäßen Verfahren Ausgangssubstanzen mit Alkylsulfonyloxygruppen eingesetzt werden, sind diese vorteilhaft solche mit 1 bis 4, insbesondere 1 oder 2, Kohlenstoffatom(en).

Sofern im erfindungsgemäßen Verfahren Ausgangssubstanzen mit Arylsulfonyloxygruppen eingesetzt werden, sind diese vorteilhaft, gegebenenfalls durch 1 oder mehr Methylrest(e) substituierte, Phenylsulfonyloxygruppen.

Vorzugsweise wird bei der Variante a) des erfindungsgemäßen Verfahrens in inerten organischen Lösungsmitteln gearbeitet.

Bei der Durchführung des erfindungsgemäßen Verfahrens a) wird eine Verbindung der allgemeinen Formel (II) — worin $R_1$, $R_2$, $R_3$, $R_6$ die obige Bedeutung haben und Y für eine Gruppe der Formel —OM (worin M die bereits angegebene Bedeutung hat) steht — in einem wasserfreien, für die Reaktion neutralen, organischen Lösungsmittel mit einem Piperazin-Derivat der allgemeinen Formel (III) — worin $R_4$, $R_5$ und n die obige Bedeutung haben und X für Halogenatom, vorzugsweise Chlor- oder Bromatom, steht — umgesetzt. Als Lösungsmittel können beispielsweise aliphatische oder acyclische Äther, wie Äthyläther, Tetrahydrofuran, Dioxan, aliphatische oder aromatische Kohlenwasserstoffe, n-Hexan, Ligroin, Benzol, Toluol, Xylol oder Dimethylsulfoxyd, Hexamethyl-phosphoramid oder ein Gemisch dieser Lösungsmittel verwendet werden. Die Reaktion wird vorzugsweise in inerter Gasatmosphäre, zum Beispiel in Stickstoff oder Argon, durchgeführt.

Wenn in der verwendeten Verbindung der allgemeinen Formel (II) Y für Halogenatom, vorzugsweise Chlor- oder Bromatom, steht, und in der verwendeten Verbindung der allgemeinen Formel (III) X für eine Gruppe der Formel —OM' — worin M' Alkalimetall, vorzugsweise Lithium-, Kalium- oder Natriumatom, ist — steht, werden die Reaktionsbedingungen wie oben beschrieben gewählt.

Für den Fall, dass von den Substituenten X und Y der eine eine Hydroxylgruppe und der andere ein Halogenatom ist, wird die Reaktion in Gegenwart einer zur Bindung der bei der Reaktion freiwerdenden Säure geeigneten anorganischen oder organischen Base (zum Beispiel von Alkalimetallcarbonaten, Pyridin oder Triäthylamin) durchgeführt, es kann aber auch ein Überschuss der Verbindung der allgemeinen Formel (III) als Säurebindemittel verwendet werden. Die Reaktion ist in inertem organischem Lösungsmittel, aber auch ohne Lösungsmittel durchführbar.

Steht sowohl X als auch Y für eine Hydroxylgruppe, wird die Kondensation in Gegenwart von bei der Ätherbildung gebräuchlichen anorganischen oder organischen Säuren oder ihrer sauren Salze (zum Beispiel in Gegenwart von Schwefelsäure, p-Toluol-sulfonsäure oder Natriumhydrogensulfat) bei atmosphärischem oder verringertem Druck durchgeführt und das entstehende Wasser azeotropische herausdestilliert. Als Lösungsmittel können aliphatische oder aromatische Kohlenwasserstoff (zum Beispiel n-Heptan, Toluol, Xylol), aliphatische oder acyclische Äther (zum Beispiel Di-n-butyläther, Dioxan), verschiedene das Auflösen begünstigende Lösungsmittel (zum Beispiel niedere aliphatische Säureamide oder deren Gemische) verwendet werden.

Die Variante b) des erfindungsgemäßen Verfahrens wird in Gegenwart eines säurebindenden Mittels durchgeführt.

Im Sinne des erfindungsgemässen Verfahrens b) wird eine Verbindung der allgemeinen Formel (IV) — worin die Bedeutung der Substituenten die bereits angegebene ist — vorzugsweise in Form ihres Mesylats, Tosylats, Bromids oder Chlorids, mit dem Benzhydryl-piperazin der allgemeinen Formel (V) umgesetzt. Die Reaktion wird in einem inerten organischen Lösungsmittel in Gegenwart einer zur Bindung der bei der Reaktion freiwerdenden Säure geeigneten Base durchgeführt. Als Lösungsmittel können Kohlenwasserstoff (zum Beispiel Ligroin, Benzol, Toluol, Xylol), halogenierte Kohlenwasserstoffe (zum Beispiel Chloroform), Äther (zum Beispiel Di-n-butyläther, Dioxan), Alkohole (zum Beispiel Äthanol), Ester (zum Beispiel Äthylacetate), Säureamide (zum Beispiel Dimethylformamid), Ketone (zum Beispiel Aceton, Methyl-isobutyl-keton) oder ein Gemisch der aufgezählten Lösungsmittel verwendet werden. Als Säurebindemittel können anorganische oder tertiäre organische Basen (wie Alkalimetall-carbonate, -hydroxyde, Triäthylamin, Pyridin) oder ein Überschuss an Benzhydrylpiperazin der allgemeinen Formel (V) verwendet werden. Wird als Säurebindemittel eine tertiäre organische Base oder die Verbindung der allgemeinen Formel (V) selbst verwendet, so kann diese zugleich auch als Lösungsmittel dienen. Die Reaktion kann bei einer Temperatur, die zwischen 20°C und dem Siedepunkt des Lösungsmittels liegt, gegebenenfalls in Gegenwart eines Katalysators, durchgeführt werden. Als Katalysator können zum Beispiel Alkalimetalljodide in Frage kommen.

Die Variante c) des erfindungsgemäßen Verfahrens wird in Gegenwart eines säurebindenden Mittels durchgeführt.

Im Sinne des erfindungsgemässen Verfahrens c) werden die monosubstituierten Piperazine der allgemeinen Formel (VI) mit Benzhydrylhalogeniden der allgemeinen Formel (VII), vorzugsweise mit Benzhydrylchloriden oder -bromiden, unter den beim Verfahren b) beschriebenen Bedingungen umgesetzt.

Auch bei der Durchführung der Variante d) des erfindungsgemäßen Verfahrens wird in inerten organischen Lösungsmitteln gearbeitet, wobei ein Alkuminiumkomplex als Reduktionsmittel verwendet wird.

Bei der Durchführung des Verfahrens d) werden die Amide der allgemeinen Formel (VIII) mit einem Aluminiumkomplex (beispielsweise mit Lithiumaluminiumhydrid) reduziert. Die Reduktion wird in einem inerten organischen Lösungsmittel (zum Beispiel in aliphatischen oder cycloaliphatischen Äthern, wie Diäthyläther oder Tetrahydrofuran) oder in deren Gemisch in neutraler Gasatmosphäre (zum Beispiel in Stickstoff oder Argon) durchgeführt, dann wird der entstandene Komplex hydrolysiert.

Das Überführen der erfindungsgemäßen 1-ω-[bis-(Phenyl)-alkoxy]-alkyl}-4-{di-(phenyl)-methyl}-piperazine der allgemeinen Formel I Säureadditionssalze kann nach on sich bekannten Verfahrensweisen durchgeführt werden. Die Salzbildung kann beispielsweise so erfolgen, dass man zu der mit einem inerten Lösungsmittel (zum Beispiel mit Äthanol) hergestellten Lösung der Verbindung der allgemeinen Formel (I) die entsprechende Säure zugibt und das Salz vorzugsweise mit einem sich mit Wasser nicht vermischenden organischen Lösungsmittel (wie beispielsweise Diäthyläther) ausfällt.

Die Umsetzung zur Überführung der erfindungsgemäßen 1-{ω-[bis-(Phenyl)-alkoxy]-alkyl]-4-{di-(phenyl)-methyl}-piperazine der allgemeinen Formel I in quaternäre Salze kann in einem organischen Lösungsmittel, vorzugsweise in Aceton, Acetonitril, Äthanol oder in einem Gemisch dieser bei einer Temperatur, die zwischen der Raumtemperatur und dem Siedepunkt des Lösungsmittels liegt, durchgeführt werden. Das entstandene Quaternärsalz wird zum Beispiel durch Filtrieren isoliert und erforderlichenfalls durch Kristallisieren gereinigt.

Die Ausgangsstoffe sind entweder bekannt oder können analog zu den aus der Literatur bekannten Verfahren hergestellt werden.

Die Verbindungen der allgemeinen Formel (II)

worin Y für Hydroxylgruppe oder eine Gruppe der Formel

MgHlg, worin Hlg Halogenatom ist, steht — können beispielsweise aus den entsprechenden Carbonylverbindungen durch Umsetzung mit einem Grignard-Reagens nach der Methode von M. S. Kharasch und Mittarbeitern (Grignard Reactions of Nonmetallic Substances, Ed. Prentice-Hall Inc., 138—143/1954/) synthetisiert werden. Die Alkoholate der allgemeinen Formel (II) können aus den entsprechenden Alkoholen beispielsweise durch Umsetzung mit einem Alkalimetall, Alkalimetallhydrid oder -amid entsprechend den Methoden, welche in der Literatur (Houben-Weyl: Methoden der Organischen Chemie VI/2,6—34/1963/) beschrieben sind, gewonnen werden. Die Benzhydrylhalogenide der allgemeinen Formeln (II) und (III) können zum Beispiel mit den von K. E. Hamlin und Mitarbeitern (J. Am. Chem. Soc. *71*, 2731—4/1949/) und R. Baltzly und Mitarbeitern (J. Org. Chem. *14*, 775—82/1949/) beschriebenen Methoden hergestellt werden.

Die Alkohole und Halogenide der allgemeinen Formel (III) können beispielsweise gemäss der belgischen Patentschrift Nr. 523 899 (CA *53*: 18071 i) durch die Reaktion von Benzhydrylhalogeniden der allgemeinen Formel (VII) mit 1-(2-Hydroxyäthyl)- oder 1-(3-Hydroxypropyl)-piperazin und die gegebenenfalls darauffolgende Reaktion der entstandenen Alkohole mit Thionylhalogenid synthetisiert werden. Die Alkoholate der allgemeinen Formel (III) können auf die oben erwähnte Weise hergestellt werden.

Die Verbindungen der allgemeinen Formel (IV) kann man zum Beispiel durch die von Sugasawa und

Mitarbeitern beschriebene Methode (Org. Synthesis *33*, 11/1953/) erhalten. Die Verbindungen der allgemeinen Formel (IV) können zum Beispiel auch aus den entsprechenden Alkoholen durch die Reaktion derselben mit Alkylsulfonylhalogenid (zum Beispiel Methansulfonyl-chlorid) oder mit Arylsulfonylhalogenid (zum Beispiel 4-Methyl-benzolsulfonylchlorid) hergestellt werden.

Die monosubstituierten Piperazine der allgemeinen Formeln (V) und (VI) können hergestellt werden, indem man im Überschuss verwendetes Piperazin mit Halogenverbindungen der allgemeinen Formel (VII) beziehungsweise (IV) umsetzt, beispielsweise gemäss der US—PS Nr. 3 178 422 (CA *63*: 4313 e).

Die Säureamide der allgemeinen Formel (VIII) können zum Beispiel durch die Reaktion von Alkoholaten der allgemeinen Formel (II) und Piperazin-Derivaten der allgemeinen Formel (IX)

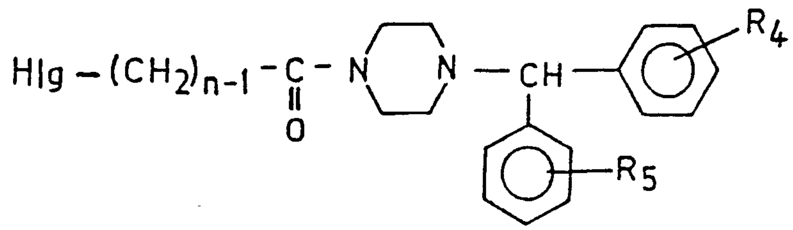

IX ,

worin die Bedeutung von $R_4$, $R_5$, n und Hlg die obige ist — gemäss der Verfahrensvariante a) hergestellt werden. Zu den Verbindungen der allgemeinen Formel (IX) kann man über die Acylierung von Benzhydrylpiperazinen der allgemeinen Formel (V) gelangen. Die Acylierung kann zum Beispiel mit 2-Chloracetylchlorid oder 3-Brompropionylchlorid in inertem organischem Lösungsmittel und in Gegenwart von Säurebindemittel, beispielsweise gemäss der US—PS Nr. 3 041 341 (CA *57*: 13778 d), durchgeführt werden.

Gegenstand der Erfindung sind auch Arzneimittel, welche 1 oder mehr der erfindungsgemäßen Verbindungen als Wirkstoff(e), gegebenenfalls zusammen mit 1 oder mehr üblichen pharmazeutischen Träger- und/oder Hilfsstoff(en), enthalten.

Die erfindungsgemäßen Verbindungen haben nämlich überlegene pharmakologische Wirkungen, insbesondere eine starke, selektive, dopaminerge Wirkung auf das Zentralnervensystem und sind als solche zur Behandlung von krankhaften Zuständen, die infolge der Degeneration und/oder Hypofunktion des dopaminergen Systems (wie zum Beispiel bestimmte neuroendokrine Erkrankungen, Depression, "Altern", Parkinsonismus und Impotenz) auftreten, geeignet. Kurz ausgedrückt handelt es sich um die Verwendung zur Behandlung von Krankheiten, die mit einer Senkung des Dopaminspiegels einhergehen.

Die dopaminerge Wirkung der erfindungsgemässen Verbindungen der allgemeinen Formel (I) bzw. ihrer Salze wurde in "in vivo"-Versuchen an Mäusen und Ratten untersucht.

a) Messen der Veränderung der locomotorischen Aktivität.

Die dopaminergen Stoffe beeinflussen die durch L-DOPA induzierte Hypermobilität (Plotnikoff N. P. und Mitarbeiter: The Thyroid Axis Drug and Behaviour, Ravan Press, N. Y. 103—113/1974/).

Für die Untersuchungen wurden 160—180 g schwere männliche Hann-Wistar-Ratten verwendet. Aus jeweils fünf Tieren bestehende Gruppen wurden intraperitoneal mit 40 mg/kg Pargylin (N-Methyl-N-/2-propinyl/-benzylamin) behandelt, dann wurden nach 60 Minuten die zu untersuchenden Stoffe in 0,5 %-iger Tween®-Suspension in einer Dosis von 30 mg/kg oral verabreicht. Die Kontrollgruppen wurden mit Placebo (0,5% Tween enthaltende destillierte wässrige Lösung, 10 ml/kg) behandelt. 30 Minuten nach Gabe der Stoffe wurden den Tieren 100 mg/kg L-DOPA intraperitoneal verabreicht. Die locomotorische Aktivität der Tiere wurde nach der Behandlung 3 Stunden lang mit einem Fünfkanal-VKI-Aktivitätsmesser (Typ: UPAMS®-01) registriert. (Während der Messzeit wurde die Dauer der aktiven Bewegung der Tiere registriert). Die Ergebnisse sind in % der Kontrolle in der Tabelle 1 angegeben.

Abkürzungen:
n = Anzahl der Tiere
i.p. = intraperitoneal
p.o. = oral
L-DOPA = L-β-3,4-Dihydroxyphenyl-alanin
x±SE = Durchschnittswert + Standardfehler

Die in der Tabelle vorkommenden Verbindungen sind:

A 1-[3'-⟨bis-{4''-(Fluor)-phenyl}-methoxy⟩-propyl]-4-[bis-{4'''(fluor)-phenyl}-methyl]-piperazin,

B 1-[3'-⟨{4''-(Methoxy)-phenyl}-{3'''-(trifluormethyl)-phenyl}-methoxy⟩-propyl]-4-[{4''''-(methoxy)-phenyl}-{3'''''-(trifluormethyl)-phenyl}-methyl]-piperazin,

C 1-[3'-⟨bis-{4''-(Fluor)-phenyl}-methoxy⟩-propyl]-4-{2'''-(chlor)-phenyl}-{4''''-(fluor)-phenyl}-methyl]-piperazin,

D 1-[3'-⟨Di-(phenyl)-methoxy⟩-propyl]-4-[{2''-(chlor)-phenyl}-{4'''-(fluor)-phenyl}-methyl]-piperazin,

E 1-[2'⟨bis-{4''-(Fluor)-phenyl}-methoxy⟩-äthyl]-4-[di-(phenyl)-methyl]-piperazin,

F 1-[3'-⟨bis-{4''-(Fluor)-phenyl}-methoxy⟩-propyl]-4-[{4'''-(fluor)-phenyl}-{phenyl}-methyl]-piperazin,

G 1-[3'-⟨{Di-(phenyl)}-methoxy⟩-propyl]-4-[bis-{(fluor)-phenyl}-methyl]-piperazin und

H 1-[3'-⟨{Di-(phenyl)}-methoxy⟩-propyl]-4-[{4''-(fluor)-phenyl}-{phenyl}-methyl]-piperazin.

Verbindung B findet in Form des Dimaleatsalzes, die übrigen Verbindungen finden in Form der Dihydrochloride Verwendung.

Tabelle 1

| Verbindung | Dosis mg/kg p.o. | locomotorische Aktivität Gesamtbewegung/3 h in % der Kontrolle | n |
|---|---|---|---|
| A | 30 | 158 ± 8,8 | 15 |
| B | 30 | 158 ± 9,2 | 15 |
| C | 30 | 165 ± 9,8 | 15 |
| D | 30 | 193 ± 10,3 | 15 |
| Placebo-Kontrolle | - | 100 ± 10,5 | 15 |

x ± S.E. = 1986 ± 208,5 sec.

Aus den Ergebnissen ist ersichtlich, dass die erfindungsgemässen Verbindungen die durch L-DOPA ausgelöste Hyperaktivität signifikant erhöhen und als solche in Zuständen des Dopaminmangels eine bedeutende verbessernde Wirkung zeigen.

b) Hemmen der neurotoxischen Wirkung von 1-Methyl-4-phenyl-1,2,3,6-tetrahydropyridin (MPTP)

1979 wurde über das MPTP geschrieben, dass es beim Menschen und Affen eine Degeneration der Neuronen des dopaminergen Systems verursacht (siehe Davis, G. C. und Mitarbeiter: Psychiat. Res. *1*, 249—254/1979/; Burns R. S. und Mitarbeiter: Proc. Nat. Acad. Sci. /USA/80 4546—4550/1983), später wurde die selektive, dopaminerge, schädigende Wirkung auch an Mäusen nachgewiesen (z.B. Hallman H. und Mitarbeiter: Eur. J. Pharmacol. *97*, 133—136/1984/; Pileblad E. und Mitarbeiter: Neuropharmacol. *24*,, 689—694/1985/). die durch MPTP an Versuchstieren hervorgerufene selektive, dopaminerge, schädigende Wirkung kann als analoger Vorgang zu Degenerations- und Hypofunktionserkrankungen des humanen dopaminergen Systems betrachtet werden, und ist ein geeignetes Modell zur Untersuchung von Verbindungen, die zur Behandlung von mit der krankhaften Funktion des dopaminergen Systems zusammenhängenden Krankheiten dienen können (Bradbury A. J. und Mitarbeiter: The Lancet/1985/, 1444—45; Przuntek H. und Mitarbeiter: Life Sci. *37*, 1195—1200/1985/).

Für die Untersuchungen wurden 20—25 g schwere männliche CFY-Mäuse (LATI, Gödöllo) verwendet. Die Verbindungen wurden in 1%-iger Tween® 80-Lösung homogenisiert und den Tieren in einer Dosis von 0,1 mMol/kg eine Stunde vor dem MPTP auf die in der Tabelle 2 angegebene Weise verabreicht. Das MPTP wurde den Mäusen in physiologischer Salzlösung frisch aufgelöst und in einer Dosis von 70 mg/kg, subcutan verabreicht. Die Tiere wurden 72—96 Stunden nach der Gabe des MPTP enthauptet, das Gehirn wurde schnell entfernt, in eiskalter physiologischer Salzlösung gekühlt, dann wurde das Striatum herauspräpariert und über Trockeneis eingefroren.

Es wurde das Gewicht der Gewebe (in gefrorenem Zustand) gemessen, dann wurden sie in 1 ml 0,4 n eiskalter, 0,5% $Na_2S_2O_5$, 0,25% $Na_2$EDTA und 100 ng N-Methyldopamin (Internal Standard zur Bestimmung der Catecholamine) enthaltender Perchlorsäure mit einem Ultra-Turrax®-Gerät homogenisiert. Das Homogenisat wurde 10 Minuten lang bei +4°C mit 20 000 g zentrifugiert, dann wurden dem Überstand 0,8 ml entnommen. Nach Zugabe von 20 mg aktiviertem $Al_2O_3$ wurde der pH mit 0,8 ml 0,5m Tris-Lösung auf 8 eingestellt, und die Röhrchen wurden 20 Minuten lang geschüttelt.

Man liess das Aluminiumoxyd sich absetzen, der Überstand wurde abgesaugt, dann wurde 3 mal mit 5 ml destilliertem Wasser gewaschen. Die am Aluminiumoxyd adsorbierten Catecholamine wurden mit 1 ml 0,05n Perchlorsäure eluiert. Aus einem Teil des Eluats wurde mittels Hochdruck-Flüssigkeitschromatographie mit Hilfe eines elektrochemischen Detektors Dopamin bestimmt (Laborpumpe MIM OE-320, 4×150 mm-Nucleosil 5 C-18 Analysen- und 4×20 mm-Nucleosil 5 C-18 Vorlaufsäule, mit Glassy-carbon-Arbeitselektrode und Ag/$AgCl_2$-Referenzelektrode ausgestatter elektrochemischer Detektor, Eltron®-Potentiostat, Zweikanalrecorder LKB 2110, Oxydationspotential: 600 mV, mobile Phase: 0,1 m $NaH_2PO_4$, 1 mM $Na_2$EDTA, 1 mM Octansulfonsäure, die 8,5% Acetonitril enthält; Fliessgeschwindigkeit: 1 ml/min).

Mit der angewandten Methode kann eine 50—60 %-ige Senkung des Dopaminspiegels des Striatums erreicht werden. Der Schutz der MPTP-induzierten Dopaminfreisetzung wurde wie folgt errechnet:

$$\text{Hemmung \%} = \frac{[\text{Verbindung + MPTP (behandelt)}] - [\text{MPTP (behandelt)}]}{(\text{Kontrolle}) - [\text{MPTP (behandelt)}]} \times 100$$

Als Vergleichssubstanz wurde Trihexyphenidyl-hydrochlorid in einer i.p. Dosis von 10 mg/kg verwendet. Dies ist weniger als 0,1 mMol/kg, aber bei Dosen über 10 mg/kg verendeten die Tiere.

Die Ergebnisse sind in der Tabelle 2 enthalten.

Tabelle 2

| Verbindung | Dosis mMol/kg | Art der Verabreichung | Hemmung der mit MPTP induzierten DA-Senkung % | n |
|---|---|---|---|---|
| E | 0,1 | p.o. | 100 | 7 |
| F | 0,1 | i.p. | 85 | 7 |
| G | 0,1 | p.o. | 59 | 7 |
| H | 0,1 | p.o. | 61 | 7 |
| Trihexy-phenidyl-HCl | | i.p. | 7 | 7 |

Hieraus ist ersichtlich, dass die erfindungsgemässen Verbindungen der allgemeinen Formel (I) Tieren oral und/oder intraperitoneal vor der Behandlung mit MPTP verabreicht die neurotoxische, den Dopaminspiegel senkende Wirkung des MPTP in grossem Ausmass oder völlig ausschalten können. Die Verbindungen zeigen eine günstig niedrige Toxizität. Auf Grund all dieser Tatsachen sind die erfindungsgemässen Verbindungen daher ein wertvolles therapeutisches Mittel zur Beeinflussung jener Krankheitsbilder, bei denen eine Degeneration des dopaminergen Systems oder — aus anderen Gründen — eine dopaminerge Hypofunktion besteht.

Die erfindungsgemässen Verbindugen können zu Arzneimittelpräparaten verarbeitet werden. Die Arzneimittelpräparate können oral, rektal und/oder parenteral verabreicht werden. Für die orale Verabreichung wird das Präparat zu Tabletten, Dragées oder Kapseln formuliert. Zur Herstellung oraler Präparate kann als Füllstoff zum Beispiel Milchzucker oder Stärke, als Binde- oder Granuliermittel beispielsweise Gelatine, Carboxymethylzellulose-natrium, Methylzellulose, Polyvinyl-pyrrolidon oder Stärkekleister verwendet werden. Als zerfalls-förderndes Mittel wird in erster Linie Kartoffelstärke oder mikrokristalline Zellulose zugegeben, es kann aber beispielsweise auch Ultraamylopectin oder Formaldehydcasein verwendet werden. Als Antiadhäsions- und Gleitmittel kann man zum Beispiel Talk, kolloidale Kieselsäure, Stearin, Kalzium- und Magnesiumstearat anwenden.

Die Tabletten können zum Beispiel durch feuchtes Granulieren und darauffolgendes Pressen hergestellt werden. Die miteinander vermischten Wirk- und Füllstoffe sowie gegebenenfalls ein Teil der zerfallsfördernden Zusätze werden mit einer wässrigen, alkoholischen oder wässrig-alkoholischen Lösung der Bindemittel in einer entsprechenden Apparatur granuliert, dann wird das Granulat getrocknet. Danach gibt man zu dem getrockneten Präparat die übrigen zerfallsfördernden, Gleit- und Antiadhäsionsmittel, und das Gemisch wird zu Tabletten gepresst. Gegebenenfalls werden die Tabletten zur Erleichterung der Dosierung mit einer Teilungsrille versehen. Die Tabletten können auch direkt aus dem Gemisch von Wirkstoff und geeigneten Hilfsstoffen durch Pressen hergestellt werden und gewünschtenfalls bei der Arzneimittelherstellung unter Verwendung von allgemein gebräuchlichen Schutz-, Geschmacks- bzw. Farbstoffen, wie zum Beispiel Zucker, Zellulosederivaten (Methyl- oder Athylzellulose, Carboxymethylzellulose-natrium), Polyvinyl-pyrrolidon, Kalziumphosphat, Kalziumcarbonat, Lebensmittelfarbstoffen, Lebensmittelfarblacken, Aromastoffen, Eisenoxydpigmenten, dragiert werden.

Bei der Kapselherstellung wird das Gemisch aus Wirk- und Hilfsstoffen in die Kapseln gefüllt.

Für die rektale Anwendung wird das Präparat in Form von Zäpfchen hergestellt. Das Zäpfchen enthält ausser dem Wirkstoff Trägerstoffmasse, sogenanntes Adeps pro suppositorio. Als Trägerstoffe können Pflanzenfette, wie zum Beispiel erhärtete Pflanzenöle, Triglyceride von $C_{12-18}$-Fettsäuren, vorzugsweise unter dem geschützten Namen Witepsol® bekannte Trägerstoffe, verwendet werden. Der Wirkstoff wird in der zerlassenen trägerstoffmasse homogen verteilt, und es werden Zäpfchen gegossen.

Für die parenterale Gabe wird das Präparat in Form von Injektionen hergestellt. Bei der Herstellung von Injektionslösungen werden die Wirkstoffe gegebenenfalls in Gegenwart von Lösungsvermittlern [wie beispielsweise Polyoxyäthylen-sorbitan-monolaurat, -monooleat oder -monostearat (Tween®20, Tween®60, Tween®80)] in destilliertem Wasser und/oder verschiedenen organischen Lösungsmitteln (wie beispielsweise Glykoläthern) gelöst. Die Injektionslösung kann ausserdem noch verschiedene Hilfsstoffe, und zwar Konserviermittel (wie zum Beispiel Benzylalkohol, p-Hydroxybenzoesäure-methyl- oder -propylester, Benzalkoniumchlorid oder Phenyl-mercuriborat usw.), weiterhin Antioxydantien (wie zum Beispiel Ascorbinsäure, Tocopherol, Natriumpyrosulfat) und gegebenenfalls komplexbildende Stoffe zur Bindung von Metallspuren, zum Beispiel Äthylendiamintetraacetat, weiterhin Substanzen zur pH-Einstellung und Puffersubstanzen und gegebenenfalls Localanästetica (wie zum Beispiel Lidocain) enthalten. Die das erfindungsgemässe Arzneimittel enthaltende Injektionslösung wird vor dem Füllen in die Ampullen filtriert und danach sterilisiert.

Die tägliche Dosis hängt vom Zustand des Kranken und der zu behandelnden Anomalie ab. Sie beträgt bei oraler Anwendung im allgemeinen 5—200 mg.

Das erfindungsgemässe Verfahren wird anhand der nachstehenden Beispiele veranschaulicht.

Beispiel 1

1-[2'-⟨bis-{4''-(Fluor)-phenyl}-methoxy⟩-äthyl]-4-[di-(phenyl)-methyl]-piperazin

Die Suspension von 6,6 g 4,4'-Difluorbenzhydrol, 0,9 g 80 %-iger Natriumhydriddispersion und 26,5 ml wasserfreiem Toluol wird in Argonatmosphäre unter Rühren 15 Minuten lang am Rückfluss gekocht, dann wird die mit 30 ml wasserfreiem Toluol bereitete Lösung von 9,5 g 1-(2-Chloräthyl)-4-(diphenylmethyl)-piperazin zugetropft und das Reaktionsgemisch weitere 2 Stunden gekocht. Nach Abkühlen wird Wasser zugegeben, und die Phasen werden getrennt. Die Toluollösung wird mit Wasser gewaschen, über wasserfreiem Kaliumcarbonat getrocknet und bei verringertem Druck eingedampft. Der Rückstand wird an einer Silikagelsäule chromatographiert, als Eluiermittel wird Chloroform verwendet. Nach Eindampfen der entsprechenden Fraktionen wird der Rest aus Äthanol umkristallisiert. Der Schmelzpunkt der Titelverbindung beträgt 112—113°C.

Elementaranalyse der Base, auf die Formel $C_{32}H_{32}F_2N_2O$ berechnet:

berechnet: C=77,08%, H=6,47%, F=7,62%, N=5,62%;

gefunden: C=77,12%, H=6,55%, F=7,58%, N=5,67%.

Zum Abtrennen des salzsauren Salzes wird die Base in wasserfreiem Äther gelöst und salzsaurer Äther bis zu einem pH-Wert von 2,5—3 zugegeben. Die Kristalle werden filtriert, mit Äther gewaschen und getrocknet. Der Schmelzpunkt des Dihydrochloridsalzes beträgt 185—187°C.

Beispiel 2

1-[3'-⟨1''-{2'',5''-Di-(methyl)-phenyl}-1''-(phenyl)-propoxy⟩-propyl]-4-[di-⟨phenyl⟩-methyl]-piperazin

Zu 20 ml 1,5 m Ätherlösung von Phenyl-magnesiumbromid werden unter Rühren 25 ml mit wasserfreiem Äther berreitete Lösung von 2',5'-Dimethyl-propiophenon gegeben, und das Reaktionsgemisch wird 1 Stunde lang gekocht. Danach wird eine mit 50 ml wasserfreiem Xylol bereitete Lösung von 9,8 g 1-(3-Chlorpropyl)-4-(diphenylmethyl)-piperazin zugetropft, der Äther wird herausdestilliert und das Reaktionsgemisch weitere 3 Stunden gekocht. Nach Abkühlen wird es auf Wasser gegossen, die organische Phase wird abgetrennt, mit Wasser gewaschen und mit wässriger Salzsäurelösung extrahiert. Der saure Extrakt wird mit wässriger Ammoniumhydroxydlösung alkalisch gemacht und mit Äther ausgeschüttelt. Die Ätherlösung wird über wasserfreiem Magnesiumsulfat getrocknet und eingedampft. Der Rückstand wird an einer Silikagelsäule unter Verwendung eines Gemisches von Chloroform und Methanol als Eluierungsmittel gereinigt, dann aus n-Hexan umkristallisiert. Der Schmelzpunkt der Base liegt bei 108—110°C. Elementaranalyse, auf die Formel $C_{37}H_{44}N_2O$ berechnet (Base):

berechnet: C=83,41%, H=8,33%, N=3,00%;

gefunden: C=83,60%, H=8,51%, N=3,11%.

Zur Ätherlösung der Base wird eine ätherische Maleinsäurelösung gegeben, der abgesonderte Niederschlag wird filtriert, dann getrocknet. Der Schmelzpunkt des Dihydrogenmaleatsalzes beträgt 138—140°C.

Beispiel 3

1-[3'-⟨Di-(phenyl)-methoxy⟩-propyl]-4-[{2''-(chlor)-phenyl}-{4'''-(fluor)-phenyl}-methyl]-piperazin

7,4 g Benzhydrylbromid und 21,8 g 1-(3-Hydroxypropyl)-4-[(2-chlorphenyl)-(4-fluorphenyl)-methyl]-piperazin werden in Stickstoffatmosphäre eine halbe Stunde lang auf 160—170°C gehalten, dann auf 90—100°C abgekühlt und auf Wasser gegossen. Die wäßrige Phase wird mit Benzol extrahiert, die Benzolphase mit Wasser gewaschen, über wasserfreiem Kaliumcarbonat getrocknet und bei verringertem Druck eingedampft. Das Produkt wird an einer Silikagelsäule mit eionem 4:1 Gemisch von Benzol und Chloroform als Eluierungsmittel gereinigt, dann aus n-Hexan umkristallisiert. Der Schmelzpunkt der Titelverbindung beträgt 98—99°C.

Elementaranalyse, auf die Formel $C_{33}H_{34}ClFN_2O$ berechnet (Base):
berechnet: C=74,91%, H=6,48%, Cl=6,70%, F=3,59%, H=5,29;
gefunden: C=75,00%, H=6,55%, Cl=6,65% F=3,61%, H=5,34%.

Das Dihydrochlorid der Base wird aus seiner wasserfreien Ätherlösung mit salzsaurem Äther abgetrennt, die Kristalle werden filtriert, dann getrocknet. Der Schmelzpunkt des Dihydrochlorids liegt bei 120—123°C.

Beispiel 4

1-[3'-⟨bis-{4''-(Fluor)-phenyl}-methoxy⟩-propyl]-4-[di-(phenyl)-methyl]-piperazin

10,4 g 1-[3-[bis(4-Fluorphenyl)-methoxy]propyl]-piperazin, 4,6 ml Triäthylamin und 8,1 g Benzhydrylbromid werden 8 Stunden lang in 100 ml wasserfreiem Xylol gekocht. Das Reaktionsgemisch wird nach Abkühlen mit Wasser gewaschen, über wasserfreiem Magnesiumsulfat getrocknet und bei verringertem Druck eingedampft. Der Rückstand wird aus n-Hexan umkristallisiert. Der Schmelzpunkt der Titelverbindung beträgt 78—79°C.

Elementaranalyse, auf die Formel $C_{33}H_{34}F_2N_2O$ berechnet (Base):
berechnet: C=77,31%, H=6,69%, F=7,41%, N=5,47%;
gefunden: C=77,53%, H=6,58%, F=7,43%, N=5,53%.

Der Schmelzpunkt des Dihydrochlorids beträgt 92—94°C.

Beispiel 5

1-[3'-⟨{Di-(phenyl)}-methoxy⟩-propyl]-4-[{4''-(fluor)-phenyl}-{phenyl}-methyl]-piperazindihydrochlorid

8,1 g 1-[(4-Fluorphenyl)-phenylmethyl]-piperazin, 11,8 g 3-(Diphenylmethoxy)-propylchlorid, 6,9 g pulverisiertes wasserfreies Kaliumcarbonat und 1 g Kaliumjodid werden unter Rühren in 200 ml Methylisobutylketon 18 Stunden lang unter Rückfluss gekocht. Nach Abkühlen wird das Reaktionsgemisch bei verringertem Druck eingedampft. Zum Rückstand werden Wasser und Benzol gegeben. Die organische Phase wird abgetrennt und mit Wasser gewaschen, dann nach Trocknen über wasserfreiem Magnesiumsulfat durch eine Aluminiumoxydschicht filtriert und im Vakuum eingedampft. Der Rückstand wird in wasserfreien Äther aufgenommen, und das salzsaure Salz der Titelverbindung mit salzsaurem Äther abgetrennt. Der Schmelzpunkt des Dihydrochlorids beträgt 162—165°C. Aus dem Dihydrochlorid wird die Base mit verdünnter wässriger Natriumhydroxydlösung freigesetzt. Der Schmelzpunkt des Produkts beträgt 59—60°C.

Elementaranalyse, auf die Formel $C_{33}H_{35}FN_2O$ berechnet (Base):
berechnet: C=80,13%, H=7,13%, F=3,84%, N=5,66%;
gefunden: C=80,28%, H=7,20%, F=3,90%, N=5,77%.

Analog zu dem im obigen Beispiel beschriebenen Verfahren werden die folgenden Verbindungen hergestellt:

a) 1-[3'-⟨bis-{4''-(Fluor)-phenyl}-methoxy⟩-propyl]-4-[{4'''-(fluor)-phenyl}-{phenyl}-methyl]-piperazin, Schmelzpunkt: 70—71°C. Es wird durch die Reaktion von 3-[bis(4-Fluorphenyl)-methoxy]-propyltosylat und 1-[(4-Fluorphenyl)-phenylmethyl]-piperazin hergestellt. Nach Behandlung der Ätherlösung der Base mit salzsaurem Äther erhält man das Dihydrochlorid der Verbindung, dessen Schmelzpunkt bei 147—150°C liegt.

Elementaranalyse, auf die Formel $C_{33}H_{33}F_3O$ berechnet:
berechnet: C=74,69%, H=6,27%, F=10,74%, N=5,28%;
gefunden: C=74,77%, H=6,20%, F=10,90%, N=5,43%.

b) 1-[3'-⟨{3''-(Trifluormethyl)-phenyl}-{4'''-(methoxy)-phenyl}-methoxy⟩-propyl]-4-[{3''''-(trifluormethyl)-phenyl}-{4'''''(methoxy)-phenyl}-methyl]-piperazindimaleat; Schmelzpunkt: 92—94°C. Es wird durch die Reaktion von 3-[(3-Trifluormethylphenyl)-(4-methoxyphenyl)-methoxy]-propylbromid und 1-[(3-Trifluormethylphenyl)-(4-methoxyphenyl)-methyl]-piperazin hergestellt.

Elementaranalyse der Base, auf die Formel $C_{37}H_{38}F_6N_2O_3$ berechnet:
berechnet: C=66,06%, H=5,69%, F=16,95%, N=4,16%;
gefunden: C=66,28%, H=5,75%, F=17,14%, N=4,30%.

Beispiel 6

1-[3'-⟨bis-{4''-(Fluor)-phenyl}-methoxy⟩-propyl]-4-[bis-{4'''-(fluor)-phenyl}-methyl]-piperazin

Zu 40 ml 1,0 m ätherischer Lithium-aluminium-hydridlösung wird die mit 150 ml wasserfreiem Diäthyläther bereitete Lösung von 11,3 g 1-[3-[bis(4-Fluorphenyl)-methoxypropionyl]-4-[bis(4-fluorphenylmethyl]-piperazin getropft, dann wird das Reaktionsgemisch 3 Stunden lang gekocht, abgekühlt, mit wässriger Natriumhydroxydlösung gespalten und filtriert. Die Ätherphase wird mit Wasser gewaschen, über wasserfreiem Magnesiumsulfat getrocknet und eingedampft. Der Rückstand wird in Benzol gelöst, durch eine Aluminiumoxydschicht filtriert, eingedampft, und das Produkt wird aus n-Hexan umkristallisiert. Der Schmelzpunkt der Titelverbindung liegt bei 73—74°C. Nach Lösung der Base in Äthanol und Behandlung mit salzsaurem Äther scheidet sich das Dihydrochlorid des Produktes kristallin ab, es wird filtriert und getrocknet. Der Schmelzpunkt des Salzes beträgt 175—176°C.

Elementaranalyse der Base, auf die Formel $C_{33}H_{32}F_4N_2O$ berechnet:
berechnet: C=72,24%, H=5,88%, F=13,85%, N=5,11%;
gefunden: C=72,07%, H=6,00%, F=13,88%, N=5,20%.

Beispiel 7

1-[3'-⟨{Di-(phenyl)}-methoxy⟩-propyl]-4-[bis-{(fluor)-phenyl}-methyl]-piperazin

Zu mit 100 ml Xylol bereiteter Lösung von 7,4 g Benzhydrol werden unter Rühren 12,0 g p-Toluolsulfonsäure und mit 50 ml Dimethylformamid bereitete Lösung von 10,4 g 1-[bis(4-Fluorphenylmethyl]-4-(3-hydroxypropyl)-piperazin gegeben, und das Reaktionsgemisch wird bis zum Siedepunkt erhitzt. Beim weiteren Kochen wird das bei der Kondensation entstehende Wasser azeotrop ständig herausdestilliert. Dann wird das Reaktionsgemisch gekühlt und mit verdünnter wässriger Ammoniumhydroxydlösung die Base freigesetzt. Die organische Phase wird abgetrennt, mit Wasser gewaschen, über wasserfreiem Natriumsulfat getrocknet und bei verringertem Druck eingedampft. Der Rückstand wird an einer Silikagelsäule mit einem Benzol-Chloroform-Gemisch im Verhältnis 5:1 als Eluiermittel gereinigt. Die entsprechenden Fraktionen werden eingedampft und aus n-Hexan umkristallisiert. Der Schmelzpunkt der Titelverbindung beträgt 68—69°C.

Elementaranalyse, auf die Formel $C_{33}H_{34}F_2N_2O$ berechnet (Base):
berechnet: C=77,31%, H=6,69%, F=7,41%, N=5,47%;
gefunden: C=77,53%, H=6,65%, F=7,48%, N=5,60%.

Der Schmelzpunkt des Dihydrochloridsalzes liegt bei 166—168°C.

Beispiel 8

1-[3'-⟨bis-{4''-(Fluor)-phenyl}-methoxy⟩-propyl]-4-[{2'''-(chlor)-phenyl}-{4''''-(fluor)-phenyl}-methyl]-piperazin

Die Suspension von 10,9 g 1-(3-Hydroxypropyl)-4-[(2-chlorphenyl)-(4-fluorphenyl)-methyl]-piperazin, 1,2 g 60 %-igem öligem Natriumhydrid und 50 ml wasserfreiem Xylol wird unter Rühren in Argonatmosphäre 30 Minuten lang am Rückfluss gekocht, dann wird eine mit 50 ml wasserfreiem Xylol bereitete Lösung von 8,6 g 4,4'-Difluorbenzhydrylchlorid zugetropft. Das Reaktionsgemisch wird 6 Stunden lang gekocht, dann wird nach Abkühlen Wasser zugegeben. Die organische Phase wird abgetrennt, mit Wasser gewaschen, über wasserfreiem Magnesiumsulfat getrocknet und bei verringertem Druck eingedampft. Der Rückstand wird an einer Silikagelsäule chromatographiert, mit einem Benzol-Chloroform-Gemisch im Verhältnis 5:1 eluiert, die entsprechenden Fraktionen werden vereinigt und im Vakuum eingedampft. Der Rückstand wird in wasserfreiem Äther gelöst und das Dihydrochlorid mit ätherischer Salzsäure abgetrennt. Der Schmelzpunkt des Dihydrochlorids der Titelverbindung beträgt 118—120°C.

Aus dem Dihydrochlorid wird mit verdünnter wässriger Ammoniumhydroxydlösung die Base freigesetzt und aus n-Hexan umkristallisiert. Der Schmelzpunkt der Base beträgt 91—92°C.

Nach Behandlung der Ätherlösung der Base mit der Ätherlösung von Methansulfonsäure erhält man das Dimethansulfonatsalz der Titelverbindung, dessen Schmelzpunkt bei 110—112°C liegt.

Elementaranalyse der Base, auf die Formel $C_{33}H_{32}ClF_3N_2O$ berechnet:
berechnet: C=70,14%, H=5,71%, Cl=6,27%, F=10,09%; N=4,96%;
gefunden: C=70,31%, H=5,68%, Cl=6,39% F=10,27%, N=5,08%.

Beispiel 9

1-[2'-⟨bis-{4''-(Fluor)-phenyl}-methoxy⟩-äthyl]-4-[bis-{4'''-(fluor)-phenyl}-methyl]-piperazin

12,7 g 2-[bis(4-Fluorphenyl)-methoxy]-äthylchlorid, 8,6 g 1-[bis(4-Fluorphenyl)-methyl]-piperazin, 6,2 g wasserfreies pulverisiertes Kaliumcarbonat und 0,5 g Kaliumjodid werden unter Rühren in 90 ml Methylisobutylketon 20 Stunden lang am Rückfluss gekocht. Nach Abkühlen wird das Reaktionsgemisch im Vakuum eingedampft. Zum Rückstand werden Wasser und Diäthyläther gegeben. Die Ätherphase wird mit Wasser gewaschen und nach Trocknen über Magnesiumsulfat eingedampft. Das Rohprodukt wird an einer Silikagelsäule mit einem Benzol-Chloroform-Gemisch im Verhältnis 2 : 1 als Eluiermittel gereinigt. Die entsprechenden Fraktionen werden durch Destillieren vereinigt und aus Äthanol umkristallisiert. Der Schmelzpunkt der Titelverbindung beträgt 87—88°C.

Aus der Base wird das Dihydrochlorid mit salzsaurem Äther abgetrennt. Der Schmelzpunkt des Dihydrochlorids beträgt: 198—200°C.

Elementaranalyse der Base, auf die Formel $C_{32}H_{30}F_4N_2O$ berechnet:
berechnet: C=71,89%, H=5,66%, F=14,22%, N=5,24%;
gefunden: C=72,00%, H=5,51%, F=14,40%, N=5,25%.

Beispiel 10

Aus den erfindungsgemässen Verbindungen können zum Beispiel die wie folgt zusammengesetzten Arzneimittel hergestellt werden:

Tabletten

50 g Wirkstoff, 92 g Lactose, 40 g Kartoffelstärke, 4 g Polyvinylpyrrolidon, 6 g Talk 1 g Magnesiumstearat, 1 g kolloidales Siliziumdioxyd (Aerosil) und 6 g Ultraamylopectin werden miteinander vermischt, dann werden durch Granulieren und Pressen Tabletten von 200 mg hergestellt, von denen jede einzelne 50 mg Wirkstoff enthält.

Wirkstoff: 1-[2'-⟨bis-{4''-(Fluor)-phenyl}-methoxy⟩-äthyl]-4-[di-(phenyl)-methyl]-piperazindihydrochlorid

Dragees

Die wie oben beschrieben hergestellten Tabletten werden auf bekannte Weise mit einer aus Zucker und Talk bestehenden Schicht überzogen. Die Dragees werden mit einem Gemisch aus Bienenwachs und Carnaubawachs poliert.

Drageegewicht: 250 mg.

Suspension

Zusammensetzung von 100 ml Suspension:

| | |
|---|---|
| Wirkstoff | 1,0 g |
| Natriumhydroxyd | 0,26 g |
| Zitronensäure | 0,30 g |
| Nipagin (4-Hydroxybenzoesäuremethylester) | 0,10 g |
| Carbopol®940 (Polyacrylsäure) | 0,30 g |
| Äthanol (96 %-ig) | 1,00 g |
| Himbeeraroma | 0,60 g |
| Sorbit (70 %-ige wässrige Lösung) | 71,00 g |

mit destilliertem Wasser auf 100 ml aufgefüllt.

In die mit 20 ml destilliertem Wasser bereitete Lösung von Nipagin und Zitronensäure wird unter intensivem Rühren in kleinen Portionen das Carbopol® gegeben, und die Lösung wird 10—12 Stunden lang stehengelassen. Dann werden die mit 1 ml destilliertem Wasser bereitete Lösung der obigen Menge Natriumhydroxyd, die wässrige Lösung von Sorbit und schliesslich unter Rühren die Äthanol-Himbeeraromalösung zugetropft. Zum Trägerstoff wird in kleinen Portionen der Wirkstoff gegeben, und es wird mit einem eintauchenden Homogenisator suspendiert. Schliesslich wird die Suspension mit destilliertem Wasser auf 100 ml ergänzt und der Suspensionssirup durch eine Kolloidmühle gegeben.

Wirkstoff: 1-[2'-⟨bis-{4''-(Fluor)-phenyl}-methoxy⟩-äthyl]-4-[di-(phenyl)-methyl]-piperazin.

**Patentansprüche für die Vertragsstaaten: BE CH DE FR GB IT LI LU NL SE**

1. 1-{ω-[bis-(Phenyl)-alkoxy]-alkyl}-4-{di-(phenyl)-methyl}-piperazine der allgemeinen Formel

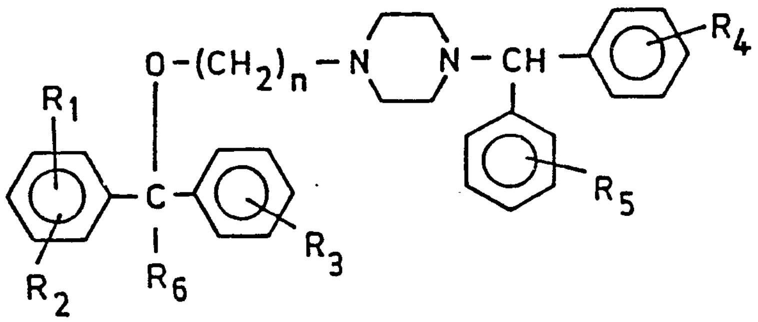

I ,

worin

$R_1$, $R_2$, $R_3$, $R_4$ und $R_5$, die gleich oder verschieden sein können, für Wasserstoffatome, Halogenatome, Trihalogenmethylreste, Alkylrest mit 1 bis 4 Kohlenstoffatom(en) oder Alkoxyreste mit 1 bis 4 Kohlenstoffatom(en) stehen,

$R_6$ ein Wasserstoffatom oder einen Alkylrest mit 1 bis 4 Kohlenstoffatom(en) bedeutet und

n 2 oder 3 ist, sowie ihre Säureadditionssalze und quaternären Salze und optisch aktiven Isomere und Solvate.

2. 1-{ω-[bis-(Phenyl)-alkoxy]-alkyl}-4-{di-(phenyl)-methyl}-piperazine nach Anspruch 1, dadurch gekennzeichnet, daß das beziehungsweise die Halogenatom(e), für welche[s] $R_1$, $R_2$, $R_3$, $R_4$ und/oder $R_5$ stehen kann beziehungsweise können, und/oder die Halogenatome des beziehungsweise der Trihalogenmethylrest[s], für welche[n] $R_1$, $R_2$, $R_3$, $R_4$ und/oder $R_5$ stehen kann beziehungsweise können, Fluor und/oder Chlor ist beziehungsweise sind.

3. 1-{ω-[bis-(Phenyl)-alkoxy]-alkyl}-4-{di-(phenyl)-methyl}-piperazine nach Anspruch 1 oder 2, dadurch gekennzeichnet, daß der beziehungsweise die Alkyl- und/oder Alkoxyrest(e), für welche[n] $R_1$, $R_2$, $R_3$, $R_4$

und/oder $R_5$ stehen kann beziehungsweise können, [ein] solche[r] mit 1 oder 2 Kohlenstoffatom(en) ist beziehungsweise sind.

4. 1-{ω-[bis-(Phenyl)-alkoxy]-alkyl}-4-{di-(phenyl)-methyl}-piperazine nach Anspruch 3, dadurch gekennzeichnet, daß der bzw. die Alkyl- und/oder Alkoxyrest(e), für welche[n] $R_1$, $R_2$, $R_3$, $R_4$ und/oder $R_5$ stehen kann bzw. können, [ein] solche[r] mit 1 Kohlenstoffatom ist bzw. sind.

5. 1-{ω-[bis-(Phenyl)-alkoxy]-alkyl}-4-{di-(phenyl)-methyl}-piperazine nach Anspruch 1 bis 4, dadurch gekennzeichnet, daß der Alkylrest, für den $R_6$ stehen kann, ein solcher mit 1 bis 3 Kohlenstoffatom(en) ist.

6. 1-[3'-⟨bis-{4''-Fluor)-phenyl}-methoxy⟩-propyl]-4-[bis-{4'''-(fluor)-phenyl}-methyl]-piperazin,

1-[3'-⟨{4''-(Methoxy)-phenyl}-{3'''-trifluormethyl)-phenyl}-methoxy⟩-propyl]-4-[{4''''-(methoxy)-phenyl}-{3'''''-(trifluormethyl)-phenyl}-methyl]-piperazin,

1-[3'-⟨bis-{4''-(Fluor)-phenyl}-methoxy⟩-propyl]-4-{2'''-(chlor)-phenyl}-{4''''-(fluor)-phenyl}-methyl]-piperazin,

1-[3'-⟨Di-(phenyl)-methoxy⟩-propyl]-4-[{2''-(chlor)-phenyl}-{4'''-(fluor)-phenyl}-methyl]-piperazin,

1-[2'⟨bis-{4''-(Fluor)-phenyl}-methoxy⟩-äthyl]-4-[di-(phenyl)-methyl]-piperazin,

1-[3'-⟨bis-{4''-(Fluor)-phenyl}-methoxy⟩-propyl]-4-[{4'''-(fluor)-phenyl}-{phenyl}-methyl]-piperazin,

1-[3'-⟨{Di-(phenyl)}-methoxy⟩-propyl]-4-[bis-{(fluor)-(phenyl)}-methyl]-piperazin und 1-[3'-⟨{Di-(phenyl)}-methoxy⟩-propyl]-4-[{4''-(fluor)-phenyl}-{phenyl}-methyl]-piperazin

sowie ihre Säureadditionssalze und quaternären Salze.

7. Verfahren zur Herstellung der Verbindungen nach Anspruch 1 bis 6, dadurch gekennzeichnet, daß man

a) Benzhydrylhalogenide, Benzhydrole beziehungsweise ihre Alkoholate der allgemeinen Formel

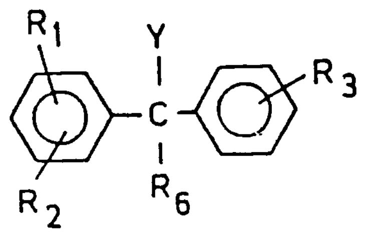

II ,

worin

$R_1$, $R_2$, $R_3$ und $R_6$ die in den Ansprüchen 1 bis 5 angegebenen Bedeutungen haben und

Y für ein Halogenatom, eine Hydroxygruppe oder einen Rest der allgemeinen Formel —OM, in welchletzterer, M ein Alkalimetallatom oder einen Rest der Formel —MgHlg, in welchletzterer Hlg ein Halogenatom ist, bedeutet, steht,

mit 1-[Di-(phenyl)-methyl]-piperazin-4-ylalk-ω-ylhalogeniden, -4-ylalkan-ω-olen beziehungsweise, -4-ylalk-ω-ylaten der allgemeinen Formel

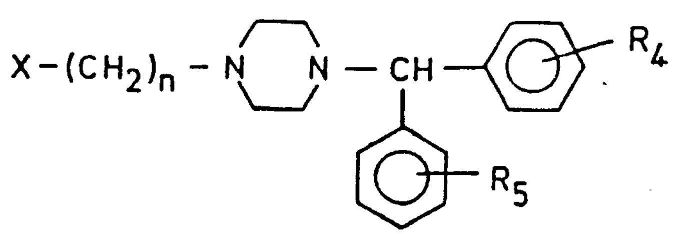

III ,

worin

$R_4$, $R_5$ und n die in den Ansprüchen 1 bis 4 angegebenen Bedeutungen haben und

X für ein Halogenatom, eine Hydroxygruppe oder einen Rest der allgemeinen Formel —OM', in welchletzterer, M' ein Alkalimetallatom bedeutet, steht, mit der Einschränkung, daß

α) X eine Hydroxygruppe oder einen Rest der allgemeinen Formel —OM', wenn Y für ein Halogenatom steht oder

β) X eine Hydroxygruppe oder ein Halogenatom bedeutet, wenn Y für eine Hydroxygruppe steht, oder

γ) X ein Halogenatom bedeutet, wenn Y für einen Rest der allgemeinen Formel —OM steht,

in einem wasserfreien, für die Reaktion neutralen organischen Lösungsmittel umsetzt, wobei man für den Fall, daß von den Substituenten X und Y der eine eine Hydroxylgruppe und der andere ein Halogen atom ist, die Reaktion in Gegenwart einer zur Bindung der bei der Reaktion frei werdenden Säure geeigneten anorganischen oder organischen Base durchführt, und für den Fall, daß sowohl X als auch Y für eine Hydroxylgruppe steht, die Kondensation in Gegenwart von bei der Ätherbildung gebräuchlichen anorganischen oder organischen Säuren oder ihren sauren Salzen durchführt und das entstehende Wasser azeotropisch herausdestilliert, oder

b) [Di-(phenyl)]-[alkyl-beziehungsweise arylsulfonyloxyalk-ω-oxy]-alkane beziehungsweise [Di-(phenyl)]-[halogenalk-ω-oxy]-alkane der allgemeinen Formel

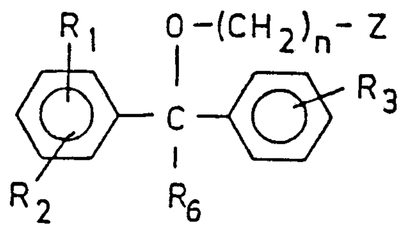

IV ,

worin

$R_1$, $R_2$, $R_3$, $R_6$ und n die in den Ansprüchen 1 bis 5 angegebenen Bedeutungen haben und

Z für einen Alkyl- oder Arylsulfonyloxyrest oder ein Halogenatom steht,

mit 1-(Benzhydryl)-piperazinen der allgemeinen Formel

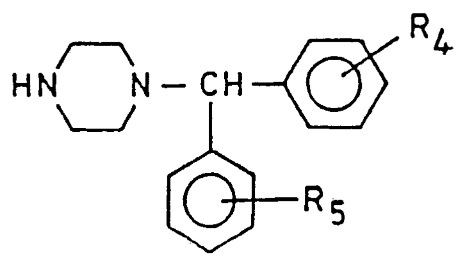

V ,

worin

$R_4$ und $R_5$ die in den Ansprüchen 1 bis 4 angegebenen Bedeutungen haben, in einem inerten organischen Lösungsmittel in Gegenwart eine zur Bindung der bei der Reaktion frei werdenden Säure geeigneten Base umsetzt oder

c) 1-{ω-[bis-(Phenyl)-alkoxy]-alkyl}-piperazine der allgemeinen Formel

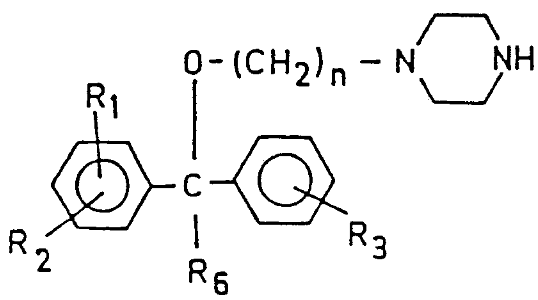

VI ,

worin

$R_1$, $R_2$, $R_3$, $R_6$ und n die in den Ansprüchen 1 bis 5 angegebenen Bedeutungen haben, mit Benzhydrylhalogenidin der allgemeinen Formel

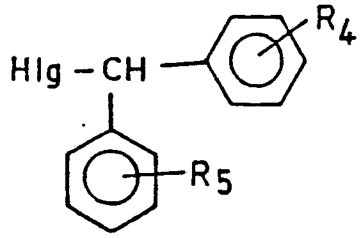

VII ,

worin

$R_4$ und $R_5$ die in den Ansprüchen 1 bis 4 angegebenen Bedeutungen haben und

Hlg die oben angegebene Bedeutung, hat, unter den vorstehend unter b) beschriebenen Bedingungen umsetzt, oder

d) 1-{ω-[bis-(Phenyl)-alkoxy]-alkanoyl}-4-{di-(phenyl)-methyl}-piperazine der allgemeinen Formel

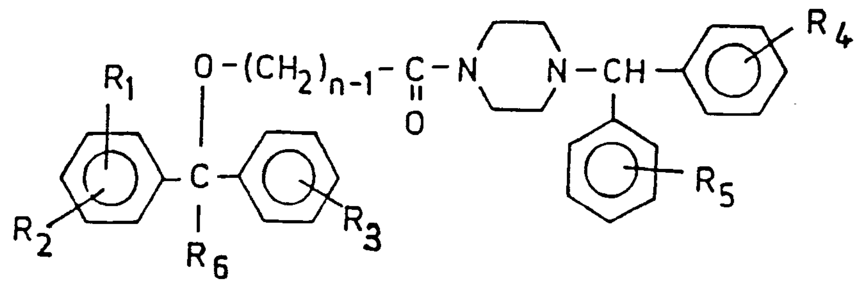

VIII ,

worin

$R_1$, $R_2$, $R_3$, $R_4$, $R_5$, $R_6$ und n die in den Ansprüchen 1 bis 5 angegebenen Bedeutungen haben, in einem inerten organischen Lösungsmittel unter Verwendung eines Aluminiumkomplexes reduziert, sowie in sich bekannter Weise gegebenenfalls die nach einer der Varianten a) bis d) erhaltenen 1-{ω-[bis-(Phenyl)-alkoxy]-alkyl}-4-{di-(phenyl)-methyl}-piperazine der allgemeinen Formel I mit anorganischen oder organischen Säuren in Säureadditionssalze oder mit Quaternisierungsmitteln in quaternäre Salze überführt und/oder die erhaltenen Säureadditionssalze oder quaternären Salze der 1-{ω-[bis-(Phenyl)-alkoxy]-alkyl}-4-{di-(phenyl)-methyl}-piperazine der allgemeinen Formel I in die entsprechenden freien 1-{ω-[bis-(Phenyl)-alkoxy]-alkyl}-4-{di-(phenyl)-methyl}-piperazinbasen der allgemeinen Formel I oder in andere Säureadditionssalze und/oder quaternäre Salze überführt und/oder gegebenenfalls eine Spaltung der erhaltenen racemischen 1-{ω-[bis-(Phenyl)-alkoxy]-alkyl}-4-{di-(phenyl)-methyl}-piperazine der allgemeinen Formel I beziehungsweise von deren Säureadditionssalzen beziehungsweise quaternären Salzen beziehungsweise eine Racemisierung der entsprechenden optisch aktiven Verbindungen in Stereoisomere vornimmt.

8. Arzneimittel, gekennzeichnet durch einen Gehalt an 1 oder mehr Verbindung(en) nach Anspruch 1 bis 6 als Wirkstoff(en), gegebenenfalls zusammen mit 1 oder mehr üblichen pharmazeutischen Träger- und/ oder Hilfsstoff(en).

**Patentansprüche für den Vertragsstaat: AT**

1. Verfahren zur Herstellung von 1-{ω-[bis-(Phenyl)-alkoxy]-alkyl}-4-{di-(phenyl)-methyl}-piperazine der allgemeinen Formel

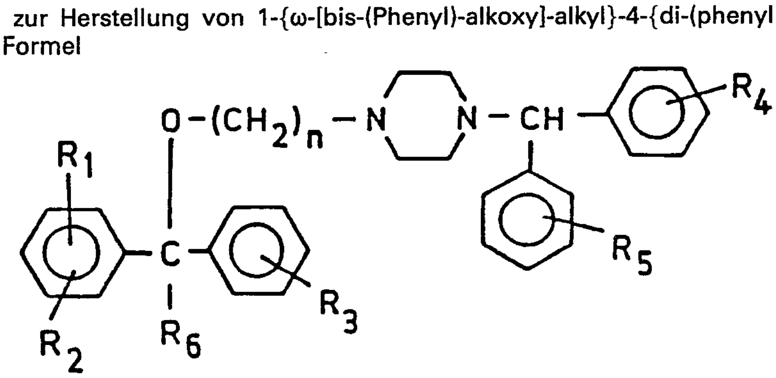

I ,

worin

$R_1$, $R_2$, $R_3$, $R_4$ und $R_5$, die gleich oder verschieden sein können, für Wasserstoffatome, Halogenatome, Trihalogenmethylreste, Alkylrest mit 1 bis 4 Kohlenstoffatom(en) oder Akoxyreste mit 1 bis 4 Kohlenstoffatom(en) stehen,

$R_6$ ein Wasserstoffatom oder einen Alkylrest mit 1 bis 4 Kohlenstoffatom(en) bedeutet und

n 2 oder 3 ist, sowie ihrer Säureadditionssalze und quaternären Salze und optisch aktiven Isomeren und Solvate, dadurch gekennzeichnet, daß man

a) Benzhydrylhalogenide, Benzhydrole beziehungsweise ihre Alkoholate der allgemeinen Formel

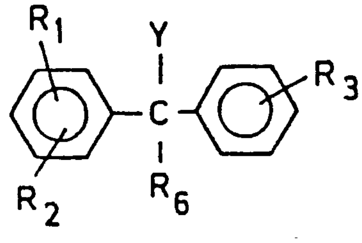

II ,

worin

$R_1$, $R_2$, $R_3$ und $R_6$ die vorstehend angegebenen Bedeutungen haben und

Y für ein Halogenatom, eine Hydroxygruppe oder einen Rest der allgemeinen Formel —OM, in welchletzterer, M ein Alkalimetallatom oder einen Rest der Formel —MgHlg, in welchletzterer Hlg ein Halogenatom ist, bedeutet, steht,

mit 1-[Di-(phenyl)-methyl]-piperazin-4-ylalk-ω-ylhalogeniden, -4-ylalkan-ω-olen beziehungsweise -4-ylalk-ω-ylaten der allgemeinen Formel

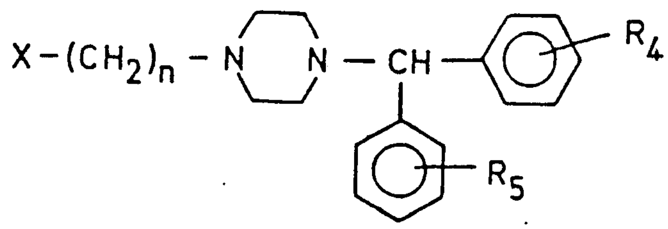

III ,

worin

$R_4$, $R_5$ und n die vorstehend angegebenen Bedeutungen haben und

X für ein Halogenatom, eine Hydroxygruppe oder einen Rest der allgemeinen Formel —OM′, in welchletzterer, M′ ein Alkalimetallatom bedeutet, steht, mit der Einschränkung, daß

α) X eine Hydroxygruppe oder einen Rest der allgemeinen Formel —OM′, wenn Y für ein Halogenatom steht oder

β) X eine Hydroxygruppe oder ein Halogenatom bedeutet, wenn Y für eine Hydroxygruppe steht, oder

γ) X ein Halogenatom bedeutet, wenn Y für einen Rest der allgemeinen Formel —OM steht,

in einem wasserfreien, für die Reaktion neutralen organischen Lösungsmittel umsetzt, wobei man für den Fall, daß von den Substituenten X und Y der eine eine Hydroxylgruppe und der andere ein Halogenatom ist, die Reaktion in Gegenwart einer zur Bindung der bei der Reaktion frei werdenden Säure geeigneten anorganischen oder organischen Base durchführt, und für den Fall, daß sowohl X als auch Y für eine Hydroxylgruppe steht, die Kondensation in Gegenwart von bei der Ätherbildung gebrächlichen anorganischen oder organischen Säuren oder ihren sauren Salzen durchführt und das entstehende Wasser azeotropisch herausdestilliert, oder

b) [Di-(phenyl)]-[alkyl-beziehungsweise arylsulfonyloxyalk-ω-oxy]-alkane beziehungsweise [Di-(phenyl)]-[halogenalk-ω-oxy]-alkane der allgemeinen Formel

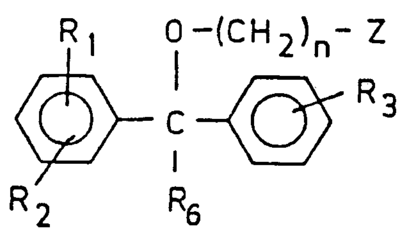

IV ,

worin

$R_1$, $R_2$, $R_3$, $R_6$ und n die vorstehende angegebenen Bedeutungen haben und

Z für einen Alkyl- oder Arylsulfonyloxyrest oder ein Halogenatom steht,

mit 1-(Benzhydryl)-piperazinen der allgemeinen Formel

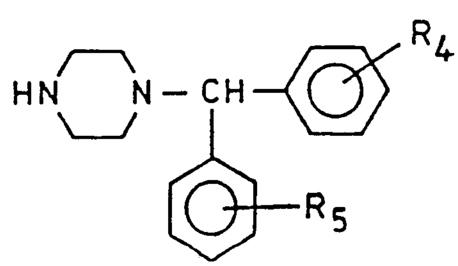

V ,

worin

$R_4$ und $R_5$ die vorstehend angegebenen Bedeutungen haben, in einem inerten organischen Lösungsmittel in Gegenwart einer zur Bindung der bei der Reaktion frei werdenden Säure geeigneten Base umsetzt oder

c) 1-{ω-[bis-(Phenyl)-alkoxy]-alkyl}-piperazine der allgemeinen Formel

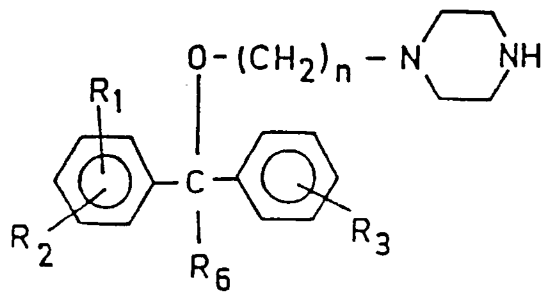

VI ,

worin

$R_1$, $R_2$, $R_3$, $R_6$ und n die vorstehend angegebenen Bedeutungen haben, mit Benzhydrylhalogenidin der allgemeinen Formel

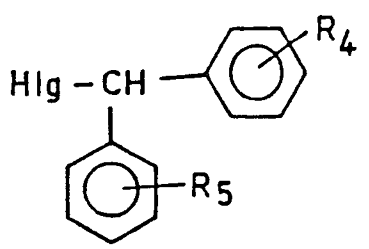

VII ,

worin

$R_4$ und $R_5$ die vorstehend angegebenen und

Hlg Bedeutungen haben und unter den vorstehend unter b) beschriebenen Bedingungen umsetzt, oder

d) 1-{ω-[bis-(Phenyl)-alkoxy]-alkanoyl}-4-{di-(phenyl)-methyl}-piperazine der allgemeinen Formel

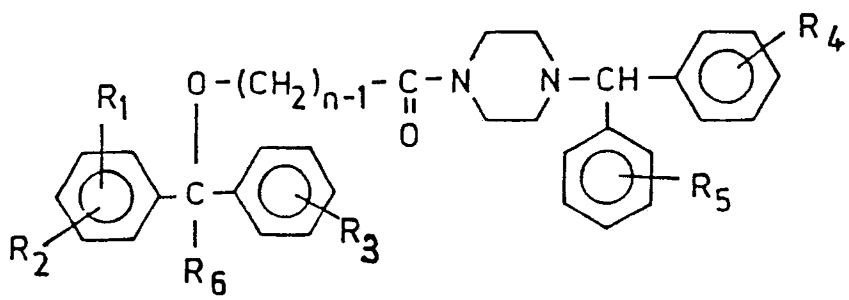

VIII ,

worin

$R_1$, $R_2$, $R_3$, $R_4$, $R_5$, $R_6$ und n die vorstehend angegebenen Bedeutungen haben,

in einem inerten organischen Lösungsmittel unter Verwendung eines Aluminiumkomplexes reduziert, sowie in an sich bekannter Weise gegebenenfalls die nach einer der Varianten a) bis d) erhaltenen 1-{ω[bis-(Phenyl)-alkoxy]-alkyl}-4-{di-(phenyl)-methyl}-piperazine der allgemeinen Formel I mit anorganischen oder organischen Säuren in Säureadditionssalze oder mit Quaternisierungsmitteln in quaternäre Salze überführt und/oder die erhaltenen Säureadditionssalze oder quaternären Salze der 1-{ω-[bis-(Phenyl)-alkoxy]-alkyl}-4-{di-(phenyl)-methyl}-piperazine der allgemeinen Formel I in die entsprechenden freien 1-{ω-[bis-(Phenyl)-alkoxy]-alkyl}-4-{di-(phenyl)-methyl}-piperazinbasen der allgemeinen Formel I oder in andere Säureadditionssalze und/oder quaternäre Salze überführt und/oder gegebenenfalls eine Spaltung der erhaltenen racemischen 1-{ω-[bis-(Phenyl)-alkoxy]-alkyl}-4-{di-(phenyl)-methyl}-piperazine der allgemeinen Formel I beziehungsweise von deren Säureadditionssalzen beziehungsweise quaternären Salzen beziehungsweise eine Racemisierung der entsprechenden optisch aktiven Verbindungen in Stereoisomere vornimmt.

2. Verfahren nach Anspruch 1, dadurch gekennzeichnet, daß solche 1-{ω-[bis-(Phenyl)-alkoxy]-alkyl}-4-{di-(phenyl)-methyl}-piperazine hergestellt werden, bei denen das beziehungsweise die Halogenatom(e), für welche[s] $R_1$, $R_2$, $R_3$, $R_4$ und/oder $R_5$ stehen kann beziehungsweise können, und/oder die Halogenatome des beziehungsweise der Trihalogenmethylrest[s], für welche[n] $R_1$, $R_2$, $R_3$, $R_4$ und/oder $R_5$ stehen kann beziehungsweise können, Fluor und/oder Chlor ist beziehungsweise sind.

3. Verfahren nach Anspruch 1 oder 2, dadurch gekennzeichnet, daß solche 1-{ω-[bis-(Phenyl)-alkoxy]-alkyl}-4-{di-(phenyl)-methyl}-piperazine hergestellt werden, bei denen der beziehungsweise die Alkyl- und/oder Alkoxyrest(e), für welche[n] $R_1$, $R_2$, $R_3$, $R_4$ und/oder $R_5$ stehen kann beziehungsweise können, [ein] solche[r] mit 1 oder 2 Kohlenstoffatom(en) ist beziehungsweise sind.

4. Verfahren nach Anspruch 3, dadurch gekennzeichnet, daß solche 1-{ω-[bis-(Phenyl)-alkoxy]-alkyl}-4-{di-(phenyl)-methyl}-piperazine hergestellt werden, bei denen der bzw. die Alkyl- und/oder Alkoxyrest(e), für welche[n] $R_1$, $R_2$, $R_3$, $R_4$ und/oder $R_5$ stehen kann bzw. können, [ein] solche[r] mit 1 Kohlenstoffatom ist bzw. sind.

5. Verfahren nach Anspruch 1 bis 4, dadurch gekennzeichnet, daß solche 1-{ω-[bis-(Phenyl)-alkoxy]-alkyl}-4-{di-(phenyl)-methyl}-piperazine hergestellt werden, bei denen der Alkylrest, für den $R_6$ stehen kann, ein solcher mit 1 bis 3 Kohlenstoffatom(en) ist.

6. Verfahren nach Anspruch 1, dadurch gekennzeichnet, daß
1-[3'-⟨bis-{4''-Fluor)-phenyl}-methoxy⟩-propyl]-4-[bis-{4'''-(fluor)-phenyl}-methyl-piperazin,
1-[3'-⟨{4''-(Methoxy)-phenyl}-{3'''-trifluormethyl)-phenyl}-methoxy⟩-propyl]-4-[{4''''-(methoxy)-phenyl}-{3'''''-(trifluormethyl)-phenyl}-methyl]-piperazin,
1-[3'-⟨bis-{4''-Fluor)-phenyl}-methoxy⟩-propyl]-4-{2'''-(chlor)-phenyl}-{4''''-(fluor)-phenyl}-methyl]-piperazin,
1-[3'-⟨Di-(phenyl)-methoxy⟩-propyl]-4-[{2''-(chlor)-phenyl}-{4'''-(fluor)-phenyl}-methyl]-piperazin,
1-[2'⟨bis-{4''-(Fluor)-phenyl}-methoxy⟩-äthyl]-4-[di-(phenyl)-methyl]-piperazin,
1-[3'-⟨bis-{4''-(Fluor)-phenyl}-methoxy⟩-propyl]-4-[{4'''-(fluor)-phenyl}-{phenyl}-methyl]-piperazin,
1-[3'-⟨{Di-(phenyl)}-methoxy⟩-propyl]-4-[bis-{(fluor)-(phenyl)}-methyl]-piperazin und 1-[3'-⟨{Di-(phenyl)}-methoxy⟩-propyl]-4-[{4''-(fluor)-phenyl}-{phenyl}-methyl]-piperazin
sowie ihre Säureadditionssalze und quaternären Salze hergestellt werden.

**Revendications pour les Etats contractants: BE CH DE FR GB IT LI LU NL SE**

1. 1-{ω-[bis-(phényl)-alcoxy]-alkyl}-4-{di-phényl)-méthyl}-pipérazines de formule générale:

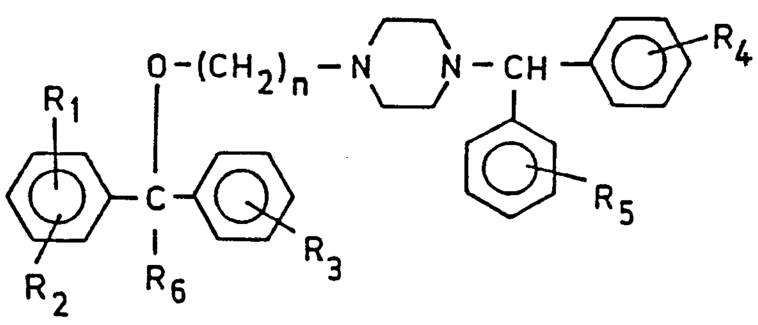

I ,

dans laquelle

$R_1$, $R_2$, $R_3$, $R_4$, et $R_5$, identiques et différents, représentent un atome d'hydrogène, d'halogène, un reste méthyle trihalogéné, un reste alkyle de 1 à 4 atomes de carbone ou un reste alcoxy de 1 à 4 atomes de carbone,

$R_6$ représente un atome d'hydrogène ou un reste alkyle de 1 à 4 atomes de carbone, et

n est égal à 2 ou 3,

ainsi que leurs sels acides d'addition, leurs sels quaternaires, leurs isomères optiquement actifs et leurs dérivés solvatés.

2. 1-{ω-[bis-(phényl)-alcoxy]-alkyl}-4-{di-phényl)-méthyl}-pipérazines selon la revendication 1, caractérisées en ce que le ou les halogènes représentent, $R_1$, $R_2$, $R_3$, $R_4$ et/ou $R_5$ et/ou celui ou ceux des restes méthyle trihalogéné représentant $R_1$, $R_2$, $R_3$, $R_4$ et/ou $R_5$ est ou sont constitué(s) par le fluor et/ou le chlore.

3. 1-{ω-[bis-(phényl)-alcoxy]-alkyl}-4-{di-phényl)-méthyl}-pipérazines selon la revendication 1 ou 2, caractérisées en ce que le ou les groupes $R_1$, $R_2$, $R_3$, $R_4$ et/ou $R_5$ peut ou peuvent être représenté(s) par un ou des restes (s) alkyle et/ou alcoxy, ce ou ces derniers comportant 1 ou 2 atomes de carbone.

4. 1-{ω-[bis-(phényl)-alcoxy]-alkyl}-4-{di-(phényl)-méthyl}-pipérazines selon la revendication 3, caractérisées en ce que les groupes $R_1$, $R_2$, $R_3$, $R_4$ et/ou $R_5$ est ou sont représenté(s) par le ou les restes alkyles et/ou alcoxy ayant 1 atome de carbone.

5. 1-{ω-[bis-(phényl)-alcoxy]-alkyl}-4-{di-(phényl)-méthyl}-pipérazines selon les revendications 1 à 4, caractérisées en ce que $R_6$ est représenté par un reste alkyle ayant 1 à 3 atomes de carbone.

6. 1-[3'-⟨bis-{4''-(fluoro)-phényl}-méthoxy⟩-propyl]-4-[bis-{4'''-(fluoro)-phényl}-méthyl-pipérazine,
1-[3'-⟨{4''-(méthoxy)-phényl}-{3'''-(trifluorométhyl)-phényl}-méthoxy⟩-propyl]-4-[{4''''-(méthoxy)-phényl}-{3'''''-(trifluorométhyl)-phényl}-methyl]-pipérazine,

1-[3'-⟨bis-{4''-(fluoro)-phényl}-méthoxy⟩-propyl]-4-{2'''-(chloro)-phényl}-{4''''-(fluoro)-phényl}-méthyl]-pipérazine,

1-[3'-⟨Di-(phényl)-méthoxy⟩-propyl]-4-[{2''-(chloro)-phényl}-{4'''-(fluoro)-phényl}-méthyl]-pipérazine,

1-[2'⟨bis-{4''-(fluoro)-phényl}-méthoxy⟩-éthyl]-4-[di-(phényl)-méthyl]-pipérazine,

1-[3'-⟨bis-{4''-(fluoro)-phényl}-méthoxy⟩-propyl]-4-[{4'''-(fluoro)-phényl}-{phényl}-méthyl]-pipérazine,

1-[3'-⟨{Di-(phényl)}-méthoxy⟩-propyl]-4-[bis-{(fluoro)-(phényl)}-méthyl]-pipérazine et

1-[3'-⟨{Di-(phényl)}-méthoxy⟩-propyl]-4-[{4''-(fluoro)-phényl}-phényl]-méthyl-pipérazine

ainsi que leurs sels acides d'addition et leurs sels quaternaires.

7. Procédé de préparaton de composés selon les revendications 1 à 6, caractérisé en ce qu'on fait réagir

a) un halogénure benzhydryle, un benzhydrole ou son alcoolate de formule générale:

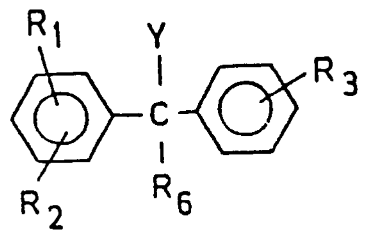

II ,

dans laquelle

$R_1$, $R_2$, $R_3$ et $R_6$ possèdent la signification indiquée dans les revendications 1 à 5 et

Y represente un atome d'halogène, un groupe hydroxy ou un reste de formule générale —OM dans laquelle M représente un métal alcalin ou un reste de formule —MgHlg, dans laquelle Hlg est un atome d'halogène, avec

le 1-[di-(phényle)-méthyle]-4-[(ω-halogène)-alkyl]-pipérazine ou le 1-[di-(phényle)-méthyle]-4-[(ω-hydroxy)-alkyl]-pipérazine ou le 1-[di-(phényle)-méthyle]-4-[(ω-alcoolate)-alkyl]-pipérazine

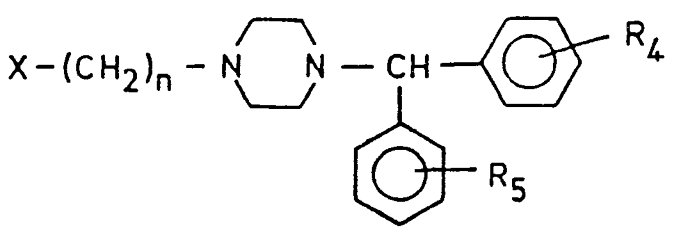

III ,

où

$R_4$, $R_5$ et n ont la signification indiquée dans les revendications 1 à 4

X représente un atome d'halogène, un groupe hydroxy ou un reste de formule générale —OM', dans lequel M' est un métal alcalin, avec la limitation en ce que:

α) X est un groupe hydroxy ou un reste de formule générale —OM', si Y est un atome d'halogène, ou bien,

β) X est un groupe hydroxy ou un atome d'halogène, si Y est un groupe hydroxy, ou bien,

γ) X est atome d'halogène, si Y est un reste de formule générale —OM,

dans un solvant organique anhydre neutre vis-à-vis de la réaction, où on conduit la réaction en présence d'une base organique ou non, convenant à la liaison d'un acide devenant libre au cours de la réaction pour le cas où un des substituants X ou Y est un groupe hydroxyle et l'autre un atome d'halogène, et on conduit la condensation en présence d'acides organiques ou non, ou bien leurs sels acides, utilisés de manière courante dans la formation d'éthers, dans le cas où X aussi bien qu'Y est un groupe hydroxyl, et on distille azéotropiquement l'eau formee, ou bien.

b) on fait réagir le di-(phényl)-[ω-alcoxy]-alcane ou le di-(phényle)-[ω-arylsulfonylalcoxy]-alcane ou le di-(phényl)-[ω-halogénoalcoxy]-alcane de formule générale

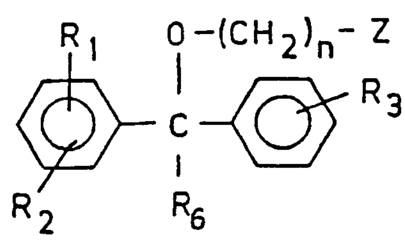

IV ,

dans laquelle

$R_1$, $R_2$, $R_3$, $R_6$ et n ont la signification indiquée dans les revendications 1 à 5, et

Z représente un reste alkyl- ou arylsulfonyloxy ou un atome d'halogène, avec, les 1-(benzhydryl)-pipérazines de formule générale:

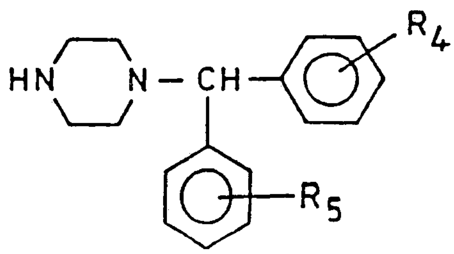

V ,

dans laquelle $R_4$ et $R_5$ ont la signification indiquée dans les revendications 1 à 4, dans un solvant organique inerte en présence d'une base convenant à la liaison d'un acide devenant libre au cours de la réaction, ou bien,

c) on fait réagir les 1-{ω-[bis-(phényl)-alkoxy]-alkyl}-pipérazines de formule générale

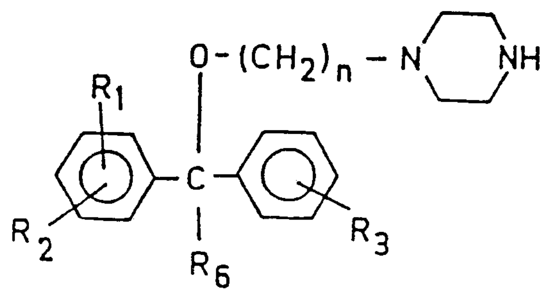

VI ,

dans laquelle

$R_1$, $R_2$, $R_3$, $R_6$ et n possèdent la signification donnée dans les revendications 1 à 5, avec les benzhydrylhalogénures de formule générale

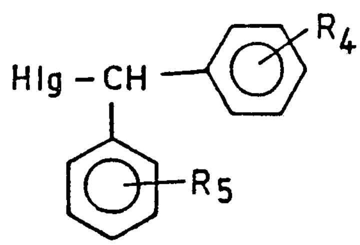

VII ,

dans laquelle

$R_4$ et $R_5$ ont les significations indiquées dans les revendications 1 à 4, et Hlg la signification donnée ci-dessus, dans les conditions indiquées ci-dessus en b) ou bien,

d) on réduit les 1-{ω-[bis-(phényl)-alkoxy]-alcanoyl}-4-{di-(phényl)-méthyl}-pipérazines de formule générale

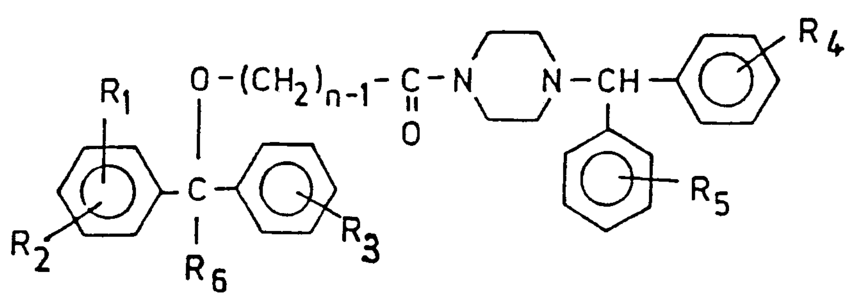

VIII ,

dans laquelle

$R_1$, $R_2$, $R_3$, $R_4$, $R_5$, $R_6$ et n ont les significations données dans les revendications 1 à 5, dans un solvant organique inerte en utilisant un complexe aluminium,

ainsi que la transformation, le cas échéant, de manière connue des 1-{ω-[bis-(phényl)-alcoxy]-alkyl}-4-{di-(phényl)-méthyl}-pipérazines de formule générale I obtenues selon l'une des variantes a) à d) en sels acides d'addition à l'aide d'acides organiques ou non, en sels quaternaires à l'aide d'un moyen de quaternisation, et/ou

la transformation des sels acides d'addition obtenus de 1-{ω-[bis-(phényl)-alcoxy]-alkyl}-4-{di-(phényl)-méthyl}-pipérazines de formule générale I en bases libres correspondantes de 1-{ω-[bis-(phényl)-alcoxy]-alkyl}-4-{di-(phényl)-méthyl}-pipérazines de formule générale I ou en d'autres sels acides d'addition et/ou sels quaternaires, et/ou

le cas échéant effecteur une séparation des 1-{ω-[bis-(phényl)-alcoxy]-alkyl}-4-{di-(phényl)-méthyl}-pipérazines racémiques obtenues de formule générale I ou de leurs sels acides d'addition ou leurs sels quaternaires, ou bien une racémisation des composés optiquement actifs obtenus en stéréoisomères.

8. Médicament, caractérisé en ce qu'il renferme un ou plusieurs composés selon les revendications 1 à 6, le cas échéant avec une ou plusieurs substances pharmaceutiques usuelles adjuvantes et/ou vectrices.

**Revendications pour l'Etat contractant: AT**

1. Procédé de préparation de 1-{ω-[bis-(phényl)-alcoxy]-alkyl}-4-{di-phényl)-méthyl}-pipérazines de formule générale:

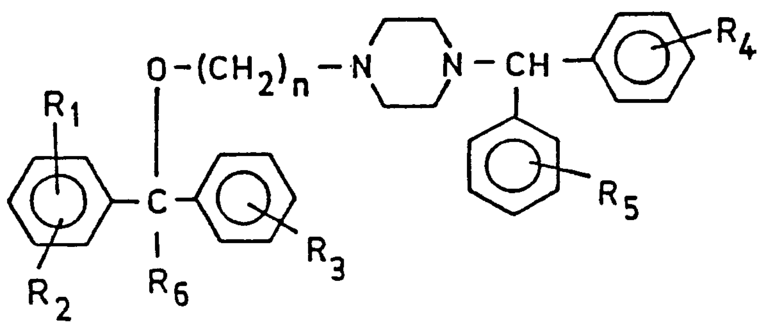

I ,

dans laquelle

$R_1$, $R_2$, $R_3$, $R_4$, et $R_5$, identiques et différents, représentent un atome d'hydrogène, d'halogène, un reste méthyle trihalogéné, un reste alkyle de 1 à 4 atomes de carbone ou un reste alcoxy de 1 à 4 atomes de carbone,

$R_6$ représente un atome d'hydrogène ou un reste alkyle de 1 à 4 atomes de carbone, et

n est égal à 2 ou 3,

ainsi que leurs sels acides d'addition, leurs sels quaternaires, leurs isomères optiquement actifs et leurs hydrates caracterisé en ce qu'on fait réagir:

a) un halogénure benzhydryle, un benzhydrole ou son alcoolate de formule générale:

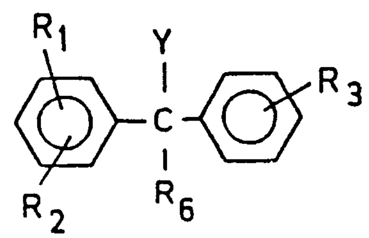

II ,

dans laquelle

$R_1$, $R_2$, $R_3$ et $R_6$ possédent la signification indiquée ci-dessus et

Y represente un atome d'halogène, un groupe hydroxy ou un reste de formule générale —OM, dans laquelle M représente un métal alcalin ou un reste de formule —MgHlg, dans laquelle Hlg est un atome d'halogène, avec

le 1-[di-(phényle)-méthyle]-4-[(ω-halogène)-alkyl]-pipérazine ou le 1-[di-(phényle)-méthyle]-4-[(ω-hydroxy)-alkyl]-pipérazine ou le 1-[di-(phényle)-méthyle]-4-[(ω-alcoolate)-alkyl]-pipérazine

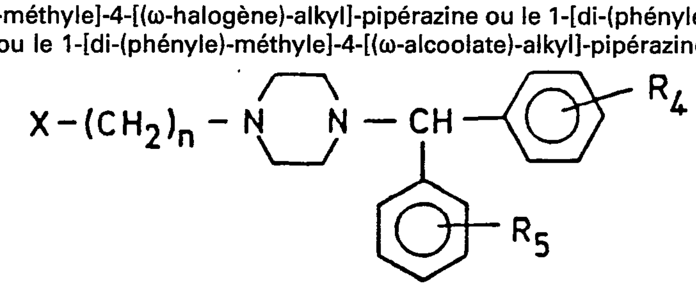

III ,

où

$R_4$, $R_5$ et n ont la signification indiquée ci-dessus,

X représente un atome d'halogène, un groupe hydroxy ou un reste de formule générale —OM', dans lequel M' est un métal alcalin, avec la limitation en ce que:

α) X est un groupe hydroxy ou un reste de formule générale —OM', si Y est un atome d'halogène, ou bien,

β) X est un groupe hydroxy ou un atome d'halogène, si Y est un groupe hydroxy, ou bien,

γ) X est atome d'halogène, si Y est un reste de formule générale —OM,

dans un solvant organique anhydre neutre vis-à-vis de la réaction, où on conduit la réaction en présence d'une base organique ou non convenant à la liaison d'un acide devenant libre au cours de la réaction pour le cas où un des substituants X ou Y est un groupe hydroxyle et l'autre un atome d'halogène, et on conduit la condensation en présence d'acides organiques ou non, ou bien leurs sels acides, utilisés de manière courante dans la formation d'éthers, dans le cas où X aussi bien qu'Y est un groupe hydroxyl, et on distille azéotropiquement l'eau formee, ou bien,

b) on fait réagir le di-(phényl)-[ω-alcoxy]-alcane ou le di-(phényle)-[ω-arylsulfonylalcoxy]-alcane ou le di-(phényl)-[ω-halogénoalcoxy]-alcane de formule générale

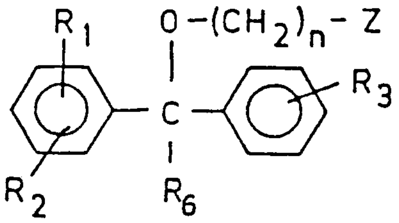

IV ,

dans laquelle

$R_1$, $R_2$, $R_3$, $R_6$ et n ont la signification indiquée ci-dessus et

Z représente un reste alkyl- ou arylsulfonyloxy ou un atome d'halogène, avec, les 1-(benzhydryl)-pipérazines de formule générale:

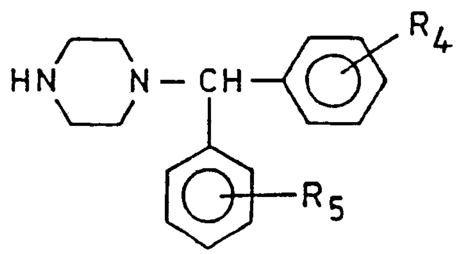

V ,

dans laquelle $R_4$ et $R_5$ ont la signification indiquée ci-dessus, dans un solvant organique inerte en présence d'une base convenant à la liaison d'un acide devenant libre au cours de la réaction, ou bien,

c) on fait réagir les 1-{ω-[bis-(phényl)-alkoxy]-alkyl}-pipérazines de formule générale

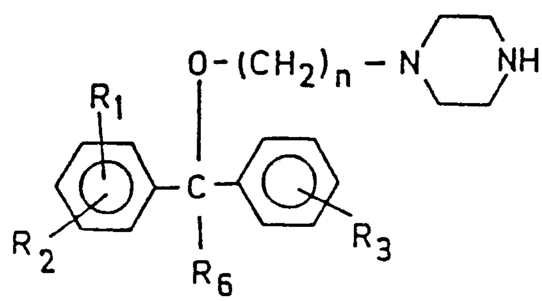

VI ,

dans laquelle

$R_1$, $R_2$, $R_3$, $R_6$ et n possèdent la signification donnée ci-dessus, avec les benzhydrylhalogénures de formule générale

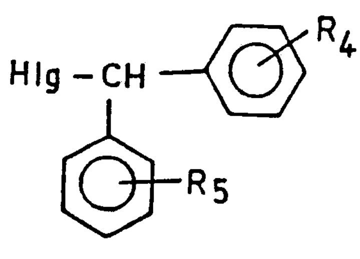

VII ,

dans laquelle

$R_4$ et $R_5$ ont les significations indiquées ci-dessus et Hlg la signification donnée ci-dessus, dans les conditions indiquées ci-dessus en b) ou bien,

d) on réduit les 1-{ω-[bis-(phényl)-alkoxy]-alkanoxy}-4-{di-(phényl)-méthyl}-pipérazines de formule générale

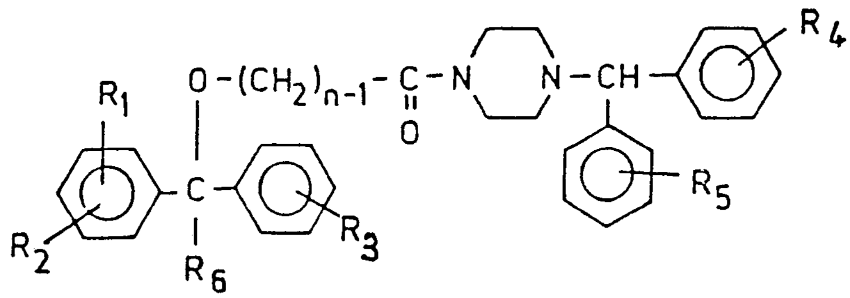

VIII ,

dans laquelle

$R_1$, $R_2$, $R_3$, $R_4$, $R_5$, $R_6$ et n ont les significations données ci-dessus dans un solvant organique inerte en utilisant un complexe d'aluminium,

ainsi que la transformation, le cas échéant, de manière connue des 1-{ω-[bis-(phényl)-alcoxy]-alkyl}-4-{di-(phényl)-méthyl}-pipérazines de formule générale I obtenues selon l'une des variantes a) à d) en sels acides d'addition à l'aide d'acides organiques ou non, en sels quaternaires à l'aide d'un moyen de quaternisation, et/ou

la transformation des sels acides d'addition obtenus de 1-{ω-[bis-(phényl)-alcoxy]-alkyl}-4-{di-(phényl)-méthyl}-pipérazines de formule générale I en bases libres correspondantes 1-{ω-[bis-(phényl)-alcoxy}-alkyl]-4-{di-(phényl)-méthyl}-pipérazines de formule générale I ou en d'autres sels acides d'addition et/ou sels quaternaires, et/ou

le cas échéant effecteur une séparation des 1-[ω-{bis-(phényl)-alcoxy}-alkyl]-4-[di-(phényl)-méthyl]-pipérazines racémiques obtenues de formule générale I ou de leurs sels acides d'addition ou leurs sels quaternaires, ou bien une racémisation des composés optiquement actifs obtenus en stéréoisomères.

2. Procédé selon la revendication 1, caractérisé en ce que les 1-{ω-[bis-(phényl)-alcoxy]-alkyl}-4-{di-(phényl)-méthyl}-pipérazines sont préparées tel(s) que le ou les halogènes représentant $R_1$, $R_2$, $R_3$, $R_4$ et/ou $R_5$ et/ou celui ou ceux des restes méthyle trihalogéné représentant $R_1$, $R_2$, $R_3$, $R_4$ et/ou $R_5$ est ou sont constitué(s) par le fluor et/ou le chlore.

3. Procédé selon la revendication 1 ou 2, caractérisé en ce que 1-{ω-[bis-(phényl)-alcoxy]-alkyl}-4-{di-(phényl)-méthyl}-pipérazines sont préparées, en ce que les groupes $R_1$, $R_2$, $R_3$, $R_4$ et/ou $R_5$ peut ou peuvent être représenté(s) par un ou des reste(s) alkyle et/ou alcoxy, ce ou ces derniers comportant 1 ou 2 atomes de carbone.

4. Procédé selon la revendication 3, caractérisé en ce que les 1-{ω-[bis-(phényl)-alcoxy]-alkyl}-4-{di-(phényl)-méthyl}-pipérazines sont préparées, en ce que le ou les groupes $R_1$, $R_2$, $R_3$, $R_4$ et/ou $R_5$ est ou sont représenté(s) par le ou les reste alkyles et/ou alcoxy ayant 1 atome de carbone.

5. Procédé selon les revendications 1 à 4, caractérisé en ce que 1-{ω-[bis-(phényl)-alcoxy]-alkyl}-4-{di-(phényl)-méthyl}-pipérazines sont préparées, en ce que $R_6$ est représenté par un reste alkyle ayant 1 à 3 atomes de carbone.

6. Procédé selon la revendication 1, caractérisé en ce que

1-[3'-⟨bis-{4''-fluoro)-phényl}-méthoxy⟩-propyl]-4-[bis-{4'''-(fluoro)-phényl}-méthyl-pipérazine,

1-[3'-⟨{4''-(méthoxy)-phényl}-{3'''-trifluorométhyl)-phényl}-méthoxy⟩-propyl]-4-[{4''''-(méthoxy)-phényl}-{3'''''-(trifluorométhyl)-phényl}-methyl]-pipérazine,

1-[3'-⟨bis-{4''-(fluoro)-phényl}-méthoxy⟩-propyl]-4-[{2'''-(chloro)-phényl}-{4''''-(fluoro)-phényl}-méthyl]-pipérazine,

1-[3'-⟨Di-(phényl)-méthoxy⟩-propyl]-4-[{2''-(chloro)-phényl}-{4'''-(fluoro)-phényl}-méthyl]-pipérazine,

1-[2'⟨bis-{4''-(fluoro)-phényl}-méthoxy⟩-éthyl]-4-[di-(phényl)-méthyl]-pipérazine,

1-[3'-⟨bis-{4''-(fluoro)-phényl}-méthoxy⟩-propyl]-4-[{4'''-(fluoro)-phényl}-{phényl}-méthyl]-pipérazine,

1-[3'-⟨{Di-(phényl)}-méthoxy⟩-propyl]-4-[bis-{(fluoro)-(phényl)}-méthyl]-pipérazine et

1-[3'-⟨{Di-(phényl)}-méthoxy⟩-propyl]-4-[{4''-(fluoro)-phényl}-{phényl}-méthyl]-pipérazine,

ainsi que leurs sels acides d'addition et leurs sels quaternaires sont preparés.

**Claims for the Contracting States: BE CH DE FR GB IT LI LU NL SE**

1. 1-{ω-[bis-(phenyl)-alkoxy]-alkyl}-4-{di-(phenyl)-methyl}-piperazines of the general formula

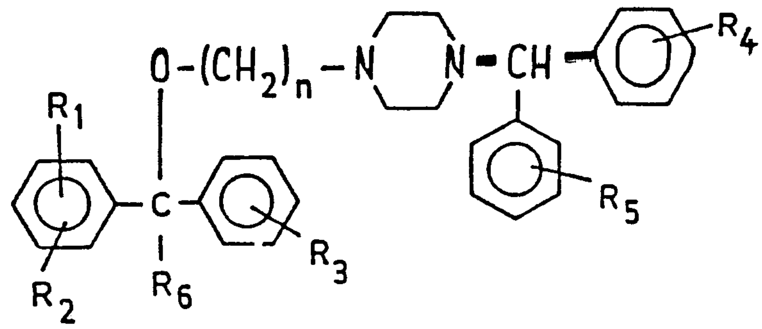

I ,

wherein

$R_1$, $R_2$, $R_3$, $R_4$ and $R_5$, which may be identical or different, represent hydrogen atoms, halogen atoms, trihalomethyl radicals, alkyl radicals having from 1 to 4 carbon atoms or alkoxy radicals having from 1 to 4 carbon atoms,

$R_6$ is a hydrogen atom or an alkyl radical having from 1 to 4 carbon atoms, and

*n* is 2 or 3,

and their acid addition salts and quaternary salts and optically active isomers and solvates.

2. 1-{ω-[bis-(phenyl)-alkoxy]-alkyl}-4-{di-(phenyl)-methyl}-piperazines according to claim 1, characterised in that the halogen atom(s) which $R_1$, $R_2$, $R_3$, $R_4$ and/or $R_5$ may represent and/or the halogen atoms of the trihalomethyl radical(s) which $R_1$, $R_2$, $R_3$, $R_4$ and/or $R_5$ may represent is(are) fluorine and/or chlorine.

3. 1-{ω-[bis-(phenyl)-alkoxy]-alkyl}-4-{di-(phenyl)-methyl}-piperazines according to claim 1 or 2, characterised in that the alkyl and/or alkoxy radical(s) which $R_1$, $R_2$, $R_3$, $R_4$ and/or $R_5$ may represent is(are) such (a) radical(s) containing 1 or 2 carbon atoms.

4. 1-{ω-[bis-(phenyl)-alkoxy]-alkyl}-4-{di-(phenyl)-methyl}-piperazines according to claim 3, characterised in that the alkyl and/or alkoxy radical(s) which $R_1$, $R_2$, $R_3$, $R_4$ and/or $R_5$ may represent is(are) such (a) radical(s) containing 1 carbon atom.

5. 1-{ω-[bis-(phenyl)-alkoxy]-alkyl}-4-{di-(phenyl)-methyl}-piperazines according to claims 1 to 4, characterised in that the alkyl radical which $R_6$ may represent is such a radical containing from 1 to 3 carbon atoms.

6. 1-[3'-⟨bis-{4''-(fluoro)-phenyl}-methoxy⟩-propyl]-4-[bis-{4'''-(fluoro)-phenyl}-methyl-piperazine,

1-[3'-⟨{4''-(methoxy)-phenyl}-{3'''-(trifluoromethyl)-phenyl}-methoxy⟩-propyl]-4-[{4''''-(methoxy)-phenyl}-{3'''''-(trifluoromethyl)-phenyl}-methyl]-piperazine,

1-[3'-⟨bis-{4''-(fluoro)-phenyl}-methoxy⟩-propyl]-4-{2'''-(chloro)-phenyl}-{4''''-(fluoro)-phenyl}-methyl]-piperazine,

1-[3'-⟨di-(phenyl)-methoxy⟩-propyl]-4-[{2''-(chloro)-phenyl}-{4'''-(fluoro)-phenyl}-methyl]-piperazine,

1-[2'-⟨bis-{4''-(fluoro)-phenyl}-methoxy⟩-ethyl]-4-[di-(phenyl)-methyl]-piperazine,

1-[3'-⟨bis-{4''-(fluoro)-phenyl}-methoxy⟩-propyl]-4-[{4'''-(fluoro)-phenyl}-{phenyl}-methyl]-piperazine,

1-[3'-⟨{di-(phenyl)}-methoxy⟩-propyl]-4-[bis-{(fluoro)-(phenyl)}-methyl]-piperazine and 1-[3'-⟨{di-(phenyl)}-methoxy⟩-propyl]-4-[{4''-(fluoro)-phenyl}-{phenyl}-methyl]-piperazine,

and their acid addition salts and quaternary salts.

7. A process for the preparation of the compounds according to claim 1 to 6, characterised in that

a) benzhydryl halides, benzhydrols or alcoholates thereof of the general formula

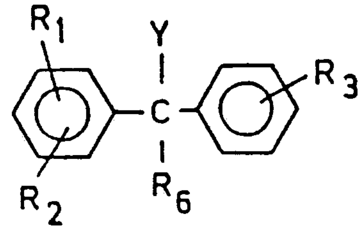

II ,

wherein

$R_1$, $R_2$, $R_3$ and $R_6$ have the meanings given in claims 1 to 5, and

Y is a halogen atom, a hydroxy group or a radical of the general formula —OM, in which M is an alkali metal atom or a radical of the formula MgHlg in which Hlg is a halogen atom,

are reacted with 1-[di-(phenyl)-methyl]-piperazin-4-ylalk-ω-yl halides, -4-ylalkan-ω-ols or -4-ylalk-ω-anolates of the general formula

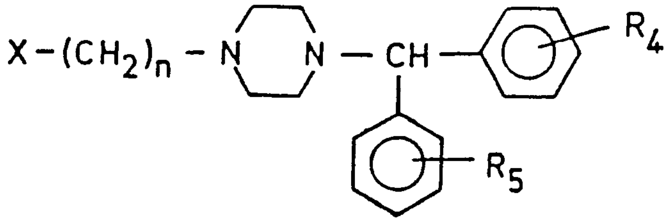

III ,

wherein

$R_4$, $R_5$ and *n* have the meanings given in claims 1 to 4, and

X is a halogen atom, a hydroxy group or a radical of the general formula —OM', wherein M' is an alkali metal atom,

with the proviso that

α) X is a hydroxy group or a radical of the general formula —OM' when Y is a halogen atom, or

β) X is a hydroxy group or a halogen atom when Y is a hydroxy group, or

γ) X is a halogen atom when Y is a radical of the general formula —OM,

in an anhydrous organic solvent that is neutral in the reaction, and in the case where one of the substituents X and Y is a hydroxy group and the other is a halogen atom, the reaction is carried out in the presence of an inorganic or organic base suitable for binding the acid freed in the reaction, and in the case where both X and Y are a hydroxy group, the condensation is carried out in the presence of inorganic or organic acids customary in ether formation, or acidic salts thereof, and the water formed is removed by azeotropic distillation, or

b) [di-(phenyl)]-[alkyl- or aryl-sulphonyloxyalk-ω-oxy]-alkanes or [di-(phenyl)]-[haloalk-ω-oxy]-alkanes of the general formula

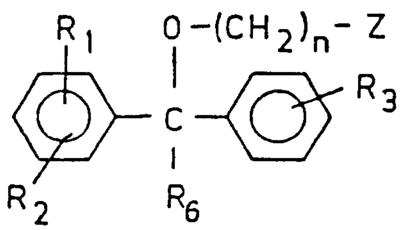

IV ,

wherein

$R_1$, $R_2$, $R_3$, $R_6$ and *n* have the meanings given in claims 1 to 5, and

Z is an alkyl- or aryl-sulphonyloxy radical or a halogen atom,

are reacted with 1-(benzhydryl)-piperazines of the general formula

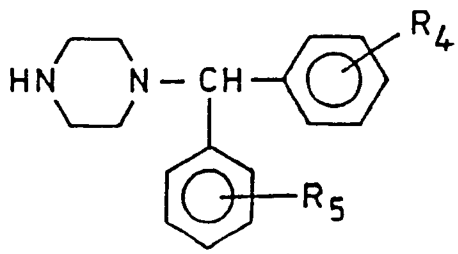

V ,

wherein

$R_4$ and $R_5$ have the meanings given in claims 1 to 4, in an inert organic solvent in the presence of a base suitable for binding the acid freed in the reaction, or

c) 1-{ω-[bis-(phenyl)-alkoxy]-alkyl}-piperazines of the general formula

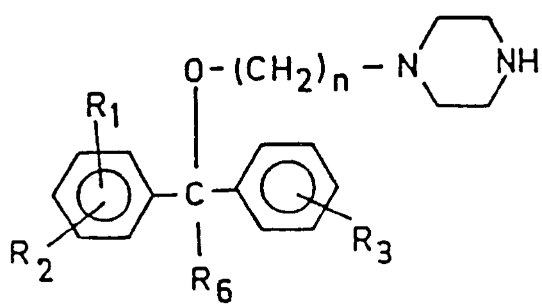

VI ,

wherein

$R_1$, $R_2$, $R_3$, $R_6$ and *n* have the meanings given in claims 1 to 5, are reacted with benzhydryl halides of the general formula

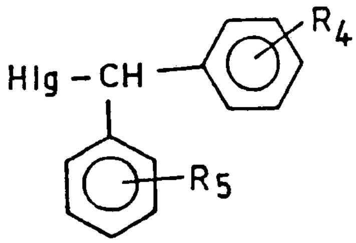

VII ,

wherein

$R_4$ and $R_5$ have the meanings given in claims 1 to 4, and Hlg has the meaning given above, under the conditions described above under b), or

d) 1-{ω-[bis-(phenyl)-alkoxy]-alkanoyl}-4-{di-(phenyl)-methyl}-piperazines of the general formula

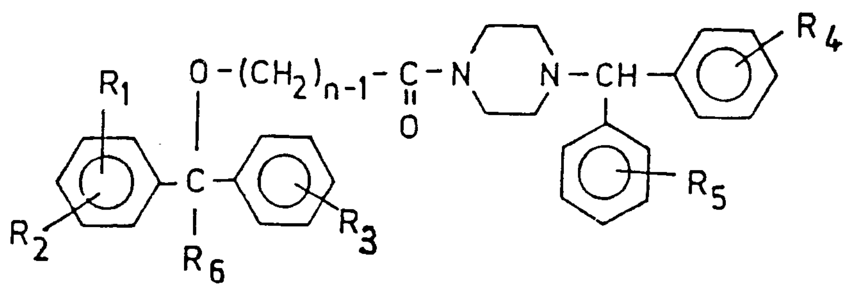

VIII ,

wherein

$R_1$, $R_2$, $R_3$, $R_4$, $R_5$, $R_6$ and *n* have the meanings given in claims 1 to 5, are reduced in an inert organic solvent using an aluminium complex,

and, in a manner known *per se*, optionally the 1-{ω-[bis-(phenyl)-alkoxy]-alkyl}-4-{di-(phenyl)-methyl}-piperazines of the general formula I obtained in accordance with one of variants a) to d) are converted into acid addition salts with inorganic or organic acids or into quaternary salts with quaternising agents and/or the resulting acid addition salts or quaternary salts of the 1-{ω-[bis-(phenyl)-alkoxy]-alkyl}-4-{di-(phenyl)-methyl}-piperazines of the general formula I are converted into the corresponding free 1-{ω-[bis-(phenyl)-alkoxy]-alkyl}-4-{di-(phenyl)-methyl}-piperazine bases of the general formula I or into other acid addition salts and/or quaternary salts and/or optionally the resulting racemic 1-{ω-[bis-(phenyl)-alkoxy]-alkyl}-4-{di-(phenyl)-methyl}-piperazines of the general formula I or their acid addition salts or quaternary salts are cleaved or the corresponding optically active compounds are racemised to form stereoisomers.

8. Medicaments, characterised by a content of one or more compounds according to claims 1 to 6 as active ingredient(s), optionally together with one or more customary pharmaceutical carriers and/or adjuncts.

**Claims for the Contracting State: AT**

1. A process for the preparation of 1-{ω-[bis-(phenyl)-alkoxy]-alkyl}-4-{di-(phenyl)-methyl}-piperazines of the general formula

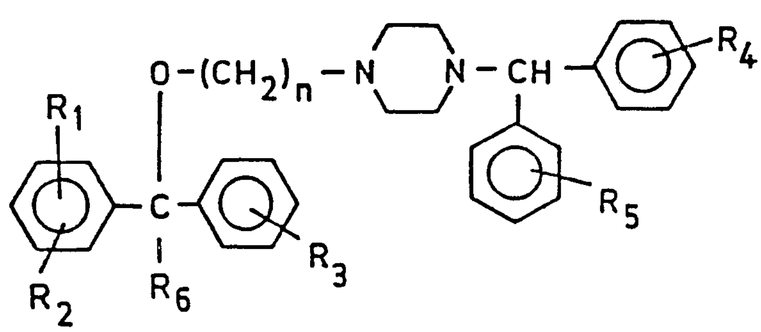

I ,

wherein

$R_1$, $R_2$, $R_3$, $R_4$ and $R_5$, which may be identical or different, represent hydrogen atoms, halogen atoms, trihalomethyl radicals, alkyl radicals having from 1 to 4 carbon atoms or alkoxy radicals having from 1 to 4 carbon atoms,

$R_6$ is a hydrogen atom or an alkyl radical having from 1 to 4 carbon atoms, and
*n* is 2 or 3,

and their acid addition salts and quaternary salts and optically active isomers and solvates characterised in that

a) benzhydryl halides, benzhydrols or alcoholates thereof of the general formula

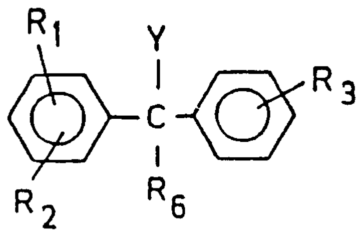

II ,

wherein

$R_1$, $R_2$, $R_3$ and $R_6$ have the meanings as defined hereinbefore,

Y is a halogen atom, a hydroxy group or a radical of the general formula —OM, in which M is an alkali metal atom or a radical of the formula —MgHlg in which Hlg is a halogen atom,

are reacted with 1-[di-(phenyl)-methyl]-piperazin-4-ylalk-ω-yl halides, -4-ylalkan-ω-ols or -4-ylalk-ω-anolates of the general formula

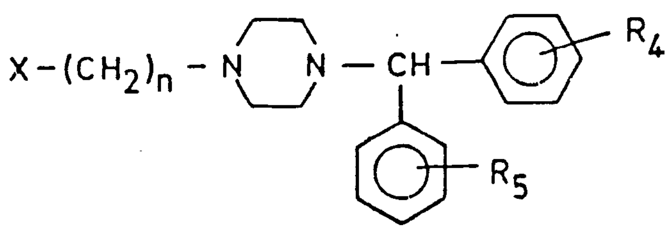

III ,

wherein

$R_4$, $R_5$ and *n* have the meanings as defined hereinbefore, and

X is a halogen atom, a hydroxy group or a radical of the general formula —OM′, wherein M′ is an alkali metal atom,

with the proviso that

α) X is a hydroxy group or a radical of the general formula —OM′ when Y is a halogen atom, or

β) X is a hydroxy group or a halogen atom when Y is a hydroxy group, or

γ) X is a halogen atom when Y is a radical of the general formula —OM,

in an anhydrous organic solvent that is neutral in the reaction, and in the case where one of the substituents X and Y is a hydroxy group and the other is a halogen atom, the reaction is carried out in the presence of an inorganic or organic base suitable for binding the acid freed in the reaction, and in the case where both X and Y are a hydroxy group, the condensation is carried out in the presence of inorganic or organic acids customary in ether formation, or acidic salts thereof, and the water formed is removed by azeotropic distillation, or

b) [di-(phenyl)]-[alkyl- or aryl-sulphonyloxyalk-ω-oxy]-alkanes or [di-(phenyl)]-[haloalk-ω-oxy]-alkanes of the general formula

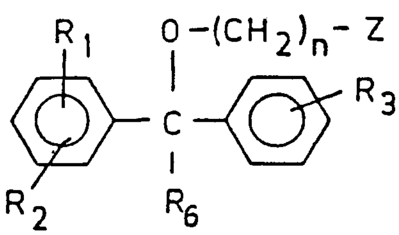

IV ,

wherein

$R_1$, $R_2$, $R_3$, $R_6$ and *n* have the meanings as defined hereinbefore,

Z is an alkyl- or aryl-sulphonyloxy radical or a halogen atom, are reacted with 1-(benzhydryl)-piperazines of the general formula

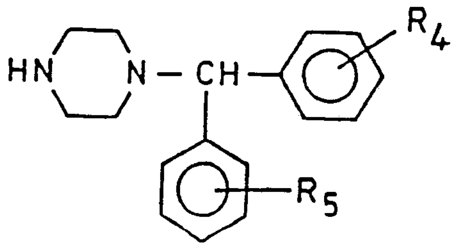

V

wherein

$R_4$ and $R_5$ have the meanings defined hereinbefore, in an inert organic solvent in the presence of a base suitable for binding the acid freed in the reaction, or

c) 1-{ω-[bis-(phenyl)-alkoxy]-alkyl}-piperazines of the general formula

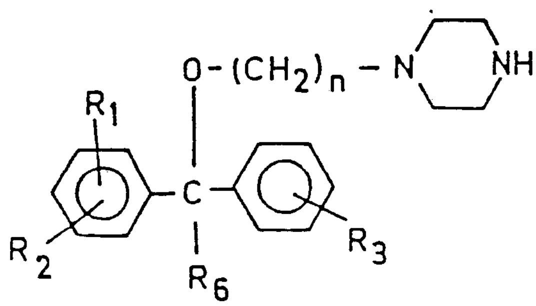

VI ,

wherein

$R_1$, $R_2$, $R_3$, $R_6$ and *n* have the meanings as defined hereinbefore, are reacted with benzhydryl halides of the general formula

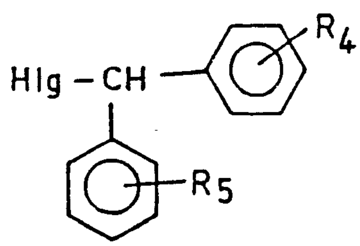

VII ,

wherein

$R_4$ and $R_5$ have the meanings as defined hereinbefore, and Hlg has the meaning given above, under the conditions described above under b), or

d) 1-{ω-[bis-(phenyl)-alkoxy]-alkanoyl}-4-{di-(phenyl)-methyl}-piperazines of the general formula

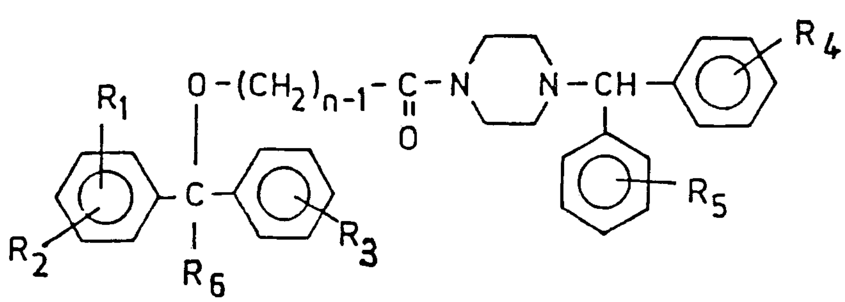

VIII ,

wherein

$R_1$, $R_2$, $R_3$, $R_4$, $R_5$, $R_6$ and *n* have the meanings as defined hereinfbefore, are reduced in an inert organic solvent using an aluminium complex, and, in a manner known *per se*, optionally the 1-{ω-[bis-(phenyl)-alkoxy]-alkyl}-4-{di-(phenyl)-methyl}-piperazines of the general formula I obtained in accordance with one of variants a) to d) are converted into acid addition salts with inorganic or organic acids or into quaternary salts with quaternising agents and/or the resulting acid addition salts or quaternary salts of the 1-{ω-[bis-(phenyl)-alkoxy]-alkyl}-4-{di-(phenyl)-methyl}-piperazines of the general formula I are converted into the corresponding free 1-{ω-[bis-(phenyl)-alkoxy]-alkyl}-4-{di-(phenyl)-methyl}-piperazine bases of the general formula I or into other acid addition salts and/or quaternary salts and/or optionally the resulting

racemic 1-{ω-[bis-(phenyl)-alkoxy]-alkyl}-4-{di-(phenyl)-methyl}-piperazines of the general formula I or their acid addition salts or quaternary salts are cleaved or the corresponding optically active compounds are racemised to form stereoisomers.

2. A process according to claim 1, characterised in that 1-{ω-[bis-(phenyl)-alkoxy]-alkyl}-4-{di-(phenyl)-methyl}-piperazines are prepared, wherein the halogen atom(s) which $R_1$, $R_2$, $R_3$, $R_4$ and/or $R_5$ may represent and/or the halogen atoms of the trihalomethyl radical(s) which $R_1$, $R_2$, $R_3$, $R_4$ and/or $R_5$ may represent is(are) fluorine and/or chlorine.

3. A process according to claim 1 or 2, characterised in that 1-{ω-[bis-(phenyl)-alkoxy]-alkyl}-4-{di-(phenyl)-methyl}-piperazines are prepared, wherein the alkyl and/or alkoxy radical(s) which $R_1$, $R_2$, $R_3$, $R_4$ and/or $R_5$ may represent is(are) such (a) radical(s) containing 1 or 2 carbon atoms.

4. A process according to claim 3, characterised in that 1-{ω-[bis-(phenyl)-alkoxy]-alkyl}-4-{di-(phenyl)-methyl}-piperazines are prepared wherein the alkyl and/or alkoxy radical(s) which $R_1$, $R_2$, $R_3$, $R_4$ and/or $R_5$ may represent is(are) such (a) radical(s) containing 1 carbon atom.

5. A process according to claims 1 to 4, characterised in that 1-{ω-[bis-(phenyl)-alkoxy]-alkyl}-4-{di-(phenyl)-methyl}-piperazines are prepared wherein the alkyl radical which $R_6$ may represent is such a radical containing from 1 to 3 carbon atoms.

6. A process according to claim 1, characterised in that

1-[3'-⟨bis-{4''-(fluoro)-phenyl}-methoxy⟩-propyl]-4-[bis-{4'''-(fluoro)-phenyl}-methyl]-piperazine,

1-[3'-⟨{4''-(methoxy)-phenyl}-{3'''-trifluoromethyl)-phenyl}-methoxy⟩-propyl]-4-[{4''''-(methoxy)-phenyl}-{3'''''-(trifluoromethyl)-phenyl}-methyl]-piperazine,

1-[3'-⟨bis-{4''-(fluoro)-phenyl}-methoxy⟩-propyl]-4-{2'''-(chloro)-phenyl}-{4''''-(fluoro)-phenyl}-methyl]-piperazine,

1-[3'-⟨di-(phenyl)-methoxy⟩-propyl]-4-[{2''-(chloro)-phenyl}-{4'''-(fluoro)-phenyl}-methyl]-piperazine,

1-[2'-⟨bis-{4''-(fluoro)-phenyl}-methoxy⟩-ethyl]-4-[di-(phenyl)-methyl]-piperazine,

1-[3'-⟨bis-{4''-(fluoro)-phenyl}-methoxy⟩-propyl]-4-[{4'''-(fluoro)-phenyl}-{phenyl}-methyl]-piperazine,

1-[3'-⟨{di-(phenyl)}-methoxy⟩-propyl]-4-[bis-{(fluoro)-(phenyl)}-methyl]-piperazine and 1-[3'-⟨{di-(phenyl)}-methoxy⟩-propyl]-4-[{4''-(fluoro)-phenyl}-{phenyl}-methyl]-piperazine, and their acid addition salts and quaternary salts are prepared.